# EUROPEAN PATENT APPLICATION

(11) **EP 4 311 859 A1**
(43) Date of publication of application: **31.01.2024**
(21) Application number: 22187293.0
(22) Date of filing: 27.07.2022
(51) Int. Cl.: C12P 5/00, C12P 17/06, C12P 7/22

(54) **PRODUCTION OF TERPENOIDS, PHENOLIC COMPOUNDS, AND VITAMINS FROM YEAST FAMILY TRICHOSPORONACEAE**

(71) Applicant: Technische Universität München, 80333 München (DE)
(72) Inventor: Masri, Mahmoud, 85221 Dachau (DE); Brück, Thomas, 85452 Eichenried (DE)
(74) Representative: Engelhard, Markus

(57) **Abstract**

The present invention relates to a method for producing at least one target compound selected from a terpenoid, a phenolic compound, a vitamin, and a combination thereof. The present invention further relates to a composition comprising at least one target compound selected from a terpenoid, a phenolic compound, a vitamin, and a combination thereof.

## Description

### FIELD OF THE INVENTION

The present invention relates to a method for producing at least one target compound selected from a terpenoid, a phenolic compound, a vitamin, and a combination thereof. The present invention further relates to a composition comprising at least one target compound selected from a terpenoid, a phenolic compound, a vitamin, and a combination thereof.

### BACKGROUND OF THE INVENTION

With the increasing world population, there is an increasing demand for valuable compounds, such as terpenoids, phenolic compounds, and vitamins. For example, squalene is a terpenoid used as a cleansing and moisturizing compound in personal care and cosmetic products including creams, lotions, lipsticks, bath oils, sunscreens, hair conditioners, and foundations. Moreover, squalene is used as an adjuvant, for example in COVID-19 vaccine formulations.

Squalene is a polyunsaturated hydrocarbon found naturally in many animals and plants, including human sebum. Squalene is a high value biological raw material having applications in various industries, which include healthcare, nutraceutical cosmetics, preparation of cosmetics as a natural moisturizer, and in the biosynthesis of cholesterol. The liver oil of deep-sea shark (cartilaginous fishes) is the first known origin for commercial purposes. Currently, supplying squalene from vegetable sources (mainly olive oil, palm oil, amaranth seeds, and wheat germs) is of great interest due to environmental concerns including the protection of marine life. For instance, olive oil has 1360-7080 mg/kg of squalene while palm oil contains 150-500 mg/kg of squalene. *Saccharomyces cerevisiae* have been genetically engineered yeast cells by the biotech company Amyris to produce commercially useful quantities of synthetic squalene with a concentration of 260 mg/kg of squalene.

In view of the growing world population, there is a need for sustainable sources of compounds of interest, such as terpenoids, phenolic compounds, and vitamins, for example, there is a need for sustainable squalene sources. Furthermore, there is a need for efficient methods of producing target compounds, such as terpenoids, phenolic compounds, and vitamins. Moreover, there is a need for producing target compounds, such as terpenoids, phenolic compounds, and vitamins, in a manner that is environmentally friendly. There is also a need for compositions comprising such target compounds, e.g. terpenoids, phenolic compounds, and/or vitamins.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method for producing at least one target compound, said method comprising the steps:
a) providing at least one yeast cell, wherein said yeast cell is a yeast from the family Trichosporonaceae; wherein, preferably, said yeast is from the genus *Cutaneotrichosporon;*
b) growing said at least one yeast cell in a medium comprising a carbon source, a nitrogen source, and a phosphate source, and thereby allowing said at least one yeast cell to produce at least one target compound;
c) optionally, lysing said at least one yeast cell;
d) harvesting said at least one target compound;
e) optionally, purifying said at least one target compound;
wherein said at least one target compound is selected from a terpenoid, a phenolic compound, a vitamin, and a combination thereof.

In one embodiment, said yeast is selected from the group consisting of *Aegeritella sp., Apiotrichum sp., Cutaneotrichosporon sp.,* and *Trichosporon sp.,* preferably selected from *Cutaneotrichosporon sp.,* more preferably is *Cutaneotrichosporon oleaginosus.*

In one embodiment, said terpenoid is selected from the group consisting of monoterpenoids, sesquiterpenoids, diterpenoids, triterpenoids, and tetraterpenoids,
wherein, optionally, triterpenoids are selected from the group consisting of squalene, 2,3-epoxysqualene, sterols, ergosterol, cholesterol, cycloartenol, stigmasterol, β-sitosterol, campesterol, fucosterol, lanosterol, steroid, oleanolic acid, glycyrrhizin, glycyrrhetinic acid, ursolic acid, betulin, betulinic acid, lupeol, and derivatives thereof, preferably selected from squalene, ergosterol, and ergosterol derivatives,
wherein, optionally, tetraterpenoids are selected from the group consisting of phytoene, xanthophylls, acyclic lycopene, and carotenoids, preferably selected from monocyclic γ-carotene and bicyclic α- and β-carotenes, more preferably selected from β-carotenes;
wherein, preferably, said terpenoid is selected from the group consisting of triterpenoids, such as squalene and sterols, e.g. ergosterol, ergosterol derivatives, cholesterol, cycloartenol, lanosterol, secosteroid, and steroid; and tetraterpenoids, such as acyclic lycopene and carotenoid, e.g. monocyclic γ-carotene and bicyclic α- and β-carotenes.

In one embodiment, said phenolic compound is selected from the group consisting of tocopherols, e.g. α-, β-, γ-, and δ-tocopherol; tocotrienols, e.g. α-, β-, γ-, and δ-tocotrienol; tocomonoenols, e.g. α- and β-tocomonoenol; phytoestrogens; chalcones, e.g. arbutin, phloretin, phloridzin, and chalconaringenin; flavonoids, e.g. flavonols, flavones, flavanones, flavanols, flavan-3-ols, anthocyanidins, isoflavones, and condensed tannins; and non-flavonoids, e.g. coumarins and phenolic acids;
wherein, preferably, said phenolic compound is selected from the group consisting of α-tocopherol, β-tocopherol, γ-tocopherol, δ-tocopherol, α-tocotrienol, β-tocotrienol, γ-tocotrienol, δ-tocotrienol, and α-tocomonoenol;
wherein, more preferably, said phenolic compound is selected from the group consisting of α-tocopherol and α-tocotrienol.

In one embodiment, said vitamin is selected from vitamin A, vitamin B₁, vitamin B₂, vitamin B₃, vitamin B₅, vitamin B₆, vitamin B₇, vitamin B₉, vitamin B₁₂, and vitamin K.

In one embodiment, said at least one target compound comprises a terpenoid and a phenolic compound; and/or
said at least one target compound is squalene, ergosterol, an ergosterol derivative, α-tocotrienol, α-tocopherol, and/or γ-tocopherol, preferably squalene, ergosterol, α-tocotrienol, α-tocopherol, and/or γ-tocopherol.

In one embodiment, said carbon source is selected from the group consisting of carbohydrates; monosaccharides, e.g. mannitol, pentoses, and hexoses, preferably glucose and xylose; oligosaccharides, e.g. maltose, lactose, sucrose, cellobiose, laminarose, cellodextrins, trehalose, raffinose, and melibiose; glycerol; amino acids; fatty acids; organic acid, e.g. acetic acid, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid, and the respective salt(s) of such acid; hydrolysates of animal tissues, plant tissues, industrial waste, food residues, or of microorganisms; and combinations of any of the foregoing; wherein, preferably, said carbon source is glucose, xylose, volatile fatty acids mix, and/or acetic acid.

In one embodiment, said industrial waste is selected from the group consisting of side stream and/or waste stream from food processing, pulp production, paper production, agro-industry, biofuel, and/or forestry.

In one embodiment, said hydrolysate(s) of microorganisms comprise or consist of microorganism's biomass, preferably selected from the group consisting of bacterial biomass, fungal biomass, yeast biomass, microalgae biomass, and combinations of any of the foregoing; wherein, preferably, said hydrolysate(s) of microorganisms comprise or consist of fungal biomass, particularly yeast biomass.

In one embodiment, said fungal biomass comprises or consists of biomass of a microorganism selected from the group consisting of *Aspergillus sp., Fusarium sp., Trichoderma sp., Ascobolus sp., Rhizopus sp.,* and combinations of any of the foregoing; wherein, preferably, said fungal biomass comprises Trichoderma reesei and /or Aspergillus niger biomass.

In one embodiment, said yeast biomass comprises or consists of biomass of a microorganism selected from the group consisting of *Saccharomyces sp., Yarrowia sp., Rhodosporidium sp., Cryptococcus sp., Trichosporon sp., Lipomyces sp., Rhodotorula sp., Candida sp., Cutaneotrichosporon sp.,* and combinations of any of the foregoing; wherein, preferably, said yeast biomass comprises *Cutaneotrichosporon oleaginosus* and/or *Saccharomyces cerevisiae* biomass.

In one embodiment, said nitrogen source is selected from the group consisting of organic nitrogen compounds, e.g. amines, amides, alkyl nitrates, nitrosamines, nitroarenes, and peroxyacyl nitrates; inorganic nitrogen compounds, e.g. ammonium salts, nitrate salts, and nitrite salts; amino acids; peptides; protein hydrolysates, e.g. peptone, tryptone, and other peptidic hydrolysates, wherein peptidic hydrolysates preferably comprise animal tissue, plant tissue, and/or components of said yeast; N-acetylglucosamine; and urea; preferably selected from the group consisting of ammonium salts, amino acids, peptides, N-acetylglucosamine, and urea.

In one embodiment, said phosphate source is selected from the group consisting of organic phosphate compounds, e.g. parathion, malathion, phospholipids, ATP, ADP, AMP, and organophosphate compounds; and inorganic phosphate salts, e.g. H₃PO₄, M₂HPO₄, MH₂PO₄, MHPO₄, and MPO₄, wherein M is a metal ion.

In one embodiment, said growing in step b) comprises a fermentative cultivation of said at least one yeast cell, wherein said at least one target compound is produced as a coproduct of said fermentative cultivation; and/or
said growing in step b) comprises cultivating said at least one yeast cell
for a period of from 1 to 7 days, preferably of from 2 to 4 days;
at a temperature in the range of from 10 °C to 45 °C, preferably in the range of from 15 °C to 40 °C, more preferably in the range of from 20 °C to 33 °C;
at a pH in the range of from 4 to 9.5, preferably in the range of from 5 to 8.5, more preferably in the range of from 5.5 to 8;
in a medium selected from minimal nitrogen media, minimal phosphate media, minimal sulfate media, and acetate rich media; and/or
with dissolved oxygen (pO₂) in a range of from 10 % to 90 %, preferably of from 20 % to 80 %, more preferably of from 30 % to 65 %.

In one embodiment, said yeast cell is a wild type yeast cell or a genetically modified yeast cell; wherein, preferably, said genetic modification comprises untargeted genetic modification, such as a chemically induced mutation or a radiation induced mutation, and/or targeted genetic modification, such as a modification using a CRISPR-CAS system, a bacterial transformation, and/or plasmid-based insertion.

In one embodiment, said harvesting in step d) comprises density-based separation, drying, floating, solvent-based extraction, chromatography, distillation, maceration, supercritical fluid extraction, enfleurage, press extraction, demulsification, decantation, and/or aspiration, preferably density-based separation,
wherein, optionally, said density-based separation is selected from separation based on natural gravity, gravity-assisted phase separation, and centrifugation, wherein each of said separation based on natural gravity, gravity-assisted phase separation, and centrifugation, is performed alone or in combination with a decantation, aspiration or other mechanical harvesting method.

In one embodiment, said harvesting in step d) comprises harvesting said at least one target compound from said medium, from a produced yeast oil, e.g. from an unsaponifiable matter of a produced yeast oil, and/or from a cell debris, preferably from an unsaponifiable matter of a produced yeast oil, optionally after a step of oil extraction;
wherein, optionally, said harvesting in step d) further comprises harvesting a compound other than the at least one target compound, preferably an oil, sugar, amino acid, and/or organic acid.

In one embodiment, said method comprises producing more than one target compound, preferably a terpenoid and a phenolic compound, more preferably at least two of squalene, ergosterol, an ergosterol derivative, α-tocotrienol, α-tocopherol, and γ-tocopherol.

In one embodiment, said lysing said at least one yeast cell in step c) comprises enzymatic hydrolysis, temperature shock, chemical treatment, high-pressure homogenization, and/or ultrasound homogenization, preferably comprises enzymatic hydrolysis.

In one embodiment, said medium used in step b) has a weight ratio of carbon to nitrogen (C:N) < 100, more preferably ≤ 80, even more preferably in a range of from 25 to 80; and/or has a weight ratio of carbon to phosphate (C:P) ≥ 10, more preferably ≥ 20, even more preferably in a range of from 25 to 100.

In one embodiment, said medium used in step b) is a limited nitrogen medium with a weight ratio of carbon to nitrogen (C:N) ≥ 80, more preferably ≥ 100, even more preferably ≥ 150; and/or is a limited phosphate medium with a weight ratio of carbon to phosphate (C:P) ≥ 100, more preferably ≥ 200, even more preferably ≥ 500; and/or is a limited sulfate medium with a weight ratio of carbon to sulfate (C:S) ≥ 10, more preferably ≥ 20, even more preferably ≥ 50.

In one embodiment, said method is performed in fed-batch manner, semi-continuous manner, or continuous mode-manner, preferably continuous mode-manner.

In one embodiment, said purifying in step e) is performed using a separation method comprising chromatography, affinity-based separation, organic solvent extraction, ionic-liquid extraction, supercritical fluid extraction, liquid-liquid extraction, solid phase extraction, flash extraction, steam extraction, vacuum distillation, distillation under inactive or noble gases, and/or deodorization.

In a further aspect, the present invention relates to a composition comprising at least one target compound selected from a terpenoid, a phenolic compound, a vitamin, and a combination thereof, wherein
the composition comprises solid biomass in a range of from 50-92 % w/w; acylglycerol(s) in a range of from 10-30 % w/w; squalene in a range of from 0.1-20 % w/w; ergosterol in a range of from 0.5-20 % w/w; free fatty acids in a range of from 0.1-0.5 % w/w; phospholipids, sphingolipids, and/or ceramides in a range of from 0.5-10 % w/w; β-carotene(s) in a range of from 0.2-3 % w/w; and tocopherol and/or tocotrienol(s) in a range of from 0.1-3 % w/w; or
the composition comprises solid biomass in a range of from 50-92 % w/w; acylglycerol(s) in a range of from 10-30 % w/w; terpenoid(s) in a range of from 3.0-30 % w/w; phenolic compound(s) in a range of from 0.5-5 % w/w; free fatty acids in a range of from 0.1-0.5 % w/w; and phospholipids, sphingolipids, and/or ceramides in a range of from 0.5-10 % w/w; or
the composition comprises solid biomass in a range of from 0-1 % w/w; acylglycerol(s) in a range of from 20-40 % w/w; squalene in a range of from 0.1-35 % w/w; ergosterol in a range of from 0.5-40 % w/w; free fatty acids in a range of from 0.1-0.5 % w/w; phospholipids, sphingolipids, and/or ceramides in a range of from 0.5-20 % w/w; β-carotene(s) in a range of from 0.2-8 % w/w; and tocopherol and/or tocotrienol(s) in a range of from 0.1-3 % w/w; or
the composition comprises solid biomass in a range of from 0-1 % w/w; acylglycerol(s) in a range of from 20-40 % w/w; terpenoid(s) in a range of from 5.0-60 % w/w; phenolic compound(s) in a range of from 0.5-10 % w/w; free fatty acids in a range of from 0.1-0.5 % w/w; and phospholipids, sphingolipids, and/or ceramides in a range of from 0.5-20 % w/w; or
the composition comprises acylglycerol(s) in a range of from 80-92 % w/w; squalene in a range of from 0.1-20 % w/w; ergosterol in a range of from 0.5-20 % w/w; free fatty acids in a range of from 0.1-0.5 % w/w; phospholipids, sphingolipids, and/or ceramides in a range of from 0.01-0.2 % w/w; β-carotene(s) in a range of from 0.2-10 % w/w; and tocopherol and/or tocotrienols in a range of from 0.1-5 % w/w; or
the composition comprises acylglycerol(s) in a range of from 80-92 % w/w; terpenoid(s) in a range of from 3.0-50 % w/w; phenolic compound(s) in a range of from 0.5-5 % w/w; free fatty acids in a range of from 0.1-0.5 % w/w; and phospholipids, sphingolipids, and/or ceramides in a range of from 0.01-1 % w/w; or
the composition comprises acylglycerol(s) in a range of from 60-92 % w/w; squalene in a range of from 0.1-10 % w/w; ergosterol in a range of from 0.5-25 % w/w; free fatty acids in a range of from 0.01-0.05 % w/w; phospholipids, sphingolipids, and/or ceramides in a range of from 0.01-0.02 % w/w; β-carotene(s) in a range of from 0.2-5 % w/w; and tocopherol and/or tocotrienols in a range of from 0.01-4 % w/w; or
the composition comprises acylglycerol(s) in a range of from 60-92 % w/w; terpenoid(s) in a range of from 3.0-40 % w/w; phenolic compound(s) in a range of from 0.5-5 % w/w; free fatty acids in a range of from 0.01-0.05 % w/w; and phospholipids, sphingolipids, and/or ceramides in a range of from 0.01-0.02 % w/w; or
the composition comprises acylglycerol(s) in a range of from 5-15 % w/w; squalene in a range of from 10-40 % w/w; ergosterol in a range of from 0.5-50 % w/w; free fatty acids in a range of from 5-15 % w/w; phospholipids, sphingolipids, and/or ceramides in a range of from 0.01-0.02 % w/w; β-carotene(s) in a range of from 0.05-15 % w/w; and tocopherol and/or tocotrienols in a range of from 0.01-15 % w/w; or
the composition of the invention comprises acylglycerol(s) in a range of from 5-15 % w/w; terpenoid(s) in a range of from 10-65 % w/w; phenolic compound(s) in a range of from 0.05-15 % w/w; free fatty acids in a range of from 5-15 % w/w; and phospholipids, sphingolipids, and/or ceramides in a range of from 0.01-0.02 % w/w.

In one embodiment, said at least one target compound has a purity of at least 1 %, 10%, 20%, 30 %, 40 %, or 50 %, preferably of at least 60 %, 70 %, or 80 %, more preferably of at least 90 %.

In one embodiment, said at least one target compound is present in said composition in a concentration of 0.1% to 50 % by weight.

In a further aspect, the present invention relates to a composition obtained by a method of the invention, as defined herein.

In one embodiment, said composition, obtained by a method of the invention, is as defined herein.

In a further aspect, the present invention relates to a composition obtainable by a method of the invention, as defined herein.

In one embodiment, said composition, obtainable by a method of the invention, is a composition as defined herein.

### DETAILED DESCRIPTION

The inventors have surprisingly found that target compound(s), particularly terpenoids, phenolic compounds, and/or vitamins can be efficiently produced with Trichosporonaceae yeasts, particularly yeasts selected from *Cutaneotrichosporon sp.,* such as *Cutaneotrichosporon oleaginosus.* The inventors have found that, unexpectedly, the method of the invention involving Trichosporonaceae yeasts, particularly yeasts selected from *Cutaneotrichosporon sp.* allows to produce target compound(s), particularly terpenoids, phenolic compounds, and/or vitamins, with high yield.

The method of the invention is highly effective in producing at least one target compound. For example, the inventors have surprisingly found that a method of the invention allows to produce at least one target compound in an amount of about 0.06 g/L/h. Thus, unexpected, the method of the invention has a very high productivity with respect to producing target compound(s). Particularly, the inventors have shown that, using the method of the invention e.g. using *Cutaneotrichosporon sp.,* target compound(s) can be produced in higher concentrations than in previously described production hosts.

The term "target compound", as used herein, relates to a compound of interest, particularly a compound obtainable using a yeast, which is selected from a terpenoid, a phenolic compound, a vitamin, and a combination thereof. In one embodiment, said at least one target compound is selected from a terpenoid, a phenolic compound, a vitamin, and a combination thereof. In one embodiment, a target compound is a terpenoid, a phenolic compound, and/or a vitamin. In a preferred embodiment, said terpenoid is a triterpenoid or tetraterpenoid. For example, a target compound may be any of vitamins A, D and K, which are terpenoids and vitamins, and vitamin E which is a phenolic compound. In one embodiment, said method comprises producing more than one target compound, e.g. any combination of a terpenoid, a phenolic compound, a vitamin, preferably at least a terpenoid and a phenolic compound. For example, the method may comprise producing at least two of a terpenoid, a phenolic compound, and a vitamin, preferably at least two of squalene, ergosterol, an ergosterol derivative, α-tocotrienol, α-tocopherol, and γ-tocopherol. In one embodiment, the term "ergosterol derivative", as used herein, relates to a compound structurally derived from ergosterol, such as provitamin D2.

In one embodiment, the terpenoid is selected from the group consisting of monoterpenoids, sesquiterpenoids, diterpenoids, triterpenoids, and tetraterpenoids. In one embodiment, the triterpenoids comprise or consist of cyclic triterpenoids, such as pentacyclic triterpenoids. In one embodiment, the triterpenoid is a cyclic triterpenoid e.g. a pentacyclic triterpenoid. In one embodiment, a pentacyclic triterpenoid is any of oleanolic acid, glycyrrhizin, glycyrrhetinic acid, ursolic acid, betulin, betulinic acid, and lupeol. In one embodiment, triterpenoids are selected from the group consisting of squalene, 2,3-epoxysqualene, sterols, ergosterol, cholesterol, cycloartenol, stigmasterol, β-sitosterol, campesterol, fucosterol, lanosterol, steroid, oleanolic acid, glycyrrhizin, glycyrrhetinic acid, ursolic acid, betulin, betulinic acid, lupeol, and derivatives thereof, preferably selected from squalene, ergosterol, and ergosterol derivatives. In one embodiment, tetraterpenoids are selected from the group consisting of phytoene, xanthophylls, acyclic lycopene, and carotenoids, preferably selected from monocyclic γ-carotene and bicyclic α- and β-carotenes, more preferably selected from β-carotenes. In a preferred embodiment, said terpenoid is selected from the group consisting of triterpenoids, such as squalene and sterols, e.g. ergosterol, ergosterol derivatives, cholesterol, cycloartenol, lanosterol, secosteroid, and steroid; and tetraterpenoids, such as acyclic lycopene and carotenoid, e.g. monocyclic γ-carotene and bicyclic α- and β-carotenes. In one embodiment, said at least one target compound comprises a terpenoid, a phenolic compound, or a combination thereof. In one embodiment, said at least one target compound comprises or consists of squalene, ergosterol, an ergosterol derivative, α-tocotrienol, α-tocopherol, and/or γ-tocopherol, preferably comprises or consists of squalene, ergosterol, α-tocotrienol, α-tocopherol, and/or γ-tocopherol. In one embodiment, said terpendoid is squalene, ergosterol, or an ergosterol derivative.

In one embodiment, said phenolic compound is selected from the group consisting of tocopherols, e.g. α-, β-, γ-, and δ-tocopherol; tocotrienols, e.g. α-, β-, γ-, and δ-tocotrienol; tocomonoenols, e.g. α- and β-tocomonoenol; phytoestrogens; chalcones, e.g. arbutin, phloretin, phloridzin, and chalconaringenin; flavonoids, e.g. flavonols, flavones, flavanones, flavanols, flavan-3-ols, anthocyanidins, isoflavones, and condensed tannins; and non-flavonoids, e.g. coumarins and phenolic acids. In one embodiment, said phenolic compound is selected from the group consisting of α-tocopherol, β-tocopherol, γ-tocopherol, δ-tocopherol, α-tocotrienol, β-tocotrienol, γ-tocotrienol, δ-tocotrienol, and α-tocomonoenol. In one embodiment, said phenolic compound is selected from the group consisting of α-tocopherol and α-tocotrienol.

In one embodiment, said vitamin is selected from vitamin A, vitamin B₁, vitamin B₂, vitamin B₃, vitamin B₅, vitamin B₆, vitamin B₇, vitamin B₉, vitamin B₁₂, and vitamin K. The vitamin produced by said yeast may be secreted vitamins are non-secreted vitamins e.g. lipid-soluble vitamins. Depending on whether the vitamin is secreted are non-secreted, the vitamin can be obtained without or prior to a cell lysis, or after cell lysis. For example, a lipid-soluble vitamin may be harvested from a microbial oil produced by said yeast.

The term "yeast", as used herein, relates to any yeast known to a person skilled in the art, e.g. an oleaginous yeast, preferably a Trichosporonaceae yeast. In one embodiment, said yeast is selected from the group consisting of *Aegeritella sp., Apiotrichum sp., Cutaneotrichosporon sp.,* and *Trichosporon sp.,* preferably selected from *Cutaneotrichosporon sp.,* more preferably is *Cutaneotrichosporon oleaginosus.* In one embodiment, said at least one yeast cell comprises a wild type yeast cell and/or a genetically modified yeast cell. In one embodiment, said at least one yeast cell comprises yeast cell(s) of more than one species, e.g. two different *Cutaneotrichosporon sp.* or one *Cutaneotrichosporon sp.* and at least one species other than *Cutaneotrichosporon sp.*

In one embodiment, the term "at least one yeast cell" relates to one or more cells of a yeast, e.g. to a plurality of cells of a species selected from Trichosporonaceae or to a plurality of cells of a mixture of two or more species selected from Trichosporonaceae. In one embodiment, said yeast is selected from the genus Cutaneotrichosporon. In one embodiment, said yeast is *Cutaneotrichosporon oleaginosus.* In a preferred embodiment, the at least one yeast cell comprises a yeast selected from *Cutaneotrichosporon* sp., preferably *Cutaneotrichosporon oleaginosus.*

In one embodiment, Trichosporonaceae are selected from Cutaneotrichosporon, Aegeritella, Apiotrichum, Effuseotrichosporon, Haglerozyma, Hyalodendron, Trichosporon, and Vanrija. In one embodiment, said yeast is selected from Cutaneotrichosporon, Aegeritella, Apiotrichum, Effuseotrichosporon, Haglerozyma, Hyalodendron, Trichosporon, and Vanrija. In one embodiment, step a) of the method of the invention comprises providing a mixture of two or more yeast species.

The term "medium", as used herein, relates to a cell culture medium comprising a carbon source, a nitrogen source, and/or a phosphate source. In one embodiment, the terms "medium" and "cell culture medium" are used interchangeably. In one embodiment, said medium comprises biomass, optionally pretreated biomass, wherein said biomass provides said carbon source, nitrogen source, and/or phosphate source. In one embodiment, said medium used in step b) has a weight ratio of carbon to nitrogen (C:N) < 100, more preferably ≤ 80, even more preferably in a range of from 25 to 80. In one embodiment, said medium used in step b) has a weight ratio of carbon to phosphate (C:P) < 10, more preferably ≤ 20, even more preferably in a range of from 25 to 500. The inventors have found that a medium having a weight ratio of carbon to nitrogen (C:N) < 100, more preferably ≤ 80, even more preferably in a range of from 25 to 80, and/or having a weight ratio of carbon to phosphate (C:P) < 10, more preferably ≤ 20, even more preferably in a range of from 25 to 500, allows to achieve a high yield of said target compound(s).

The inventors have further found that a medium used in step b) comprising or consisting of a limited nitrogen medium with a weight ratio of carbon to nitrogen (C:N) ≥ 80, more preferably ≥ 100, even more preferably ≥ 150; and/or comprising or consisting of a limited phosphate medium with a weight ratio of carbon to phosphate (C:P) ≥ 100, more preferably ≥ 200, even more preferably ≥ 500; and/or comprising or consisting of a limited sulfate medium with a weight ratio of carbon to sulfate (C:S) ≥ 10, more preferably ≥ 20, even more preferably ≥ 50, allows to achieve a high yield of said target compound(s).

In one embodiment, said method comprises producing more than one target compound by adding at least one, e.g. one or more, inducing system(s). In one embodiment, said inducing system is selected from the group consisting of squalene epoxidase (squalene monooxygenase) inhibitors, ergosterol degradation inhibitors, ergosterol biosynthesis inducers, ergosterol biosynthesis inhibitors, fatty acid synthase inhibitors, and/or a combination thereof. In one embodiment, said squalene epoxidase (squalene monooxygenase) inhibitor is selected from the group consisting of butenafine, naftifine, terbinafine, and/or a combination thereof. In one embodiment, said ergosterol biosynthesis inhibitor is selected from the group consisting of Amphotericin B, Fluconazole, miconazole, itraconazole, clotrimazole, myclobutanil, and/or a combination thereof.

In one embodiment, said carbon comprises or consists of any of carbohydrates; monosaccharides, e.g. mannitol, pentoses, and hexoses, preferably glucose and xylose; oligosaccharides, e.g. maltose, lactose, sucrose, cellobiose, laminarose, cellodextrins, trehalose, raffinose, and melibiose; glycerol; amino acids; fatty acids; organic acid, e.g. acetic acid, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid, and the respective salt(s) of such acid; hydrolysates of animal tissues, plant tissues, industrial waste, food residues, or of microorganisms; and combinations thereof. In one embodiment, said carbon source is glucose, xylose, and/or acetic acid.

In one embodiment, said industrial waste comprises or consists of side stream and/or waste stream from food processing, pulp production, paper production, agro-industry, biofuel, and/or forestry. In one embodiment, said hydrolysates of microorganisms comprise or consist of bacterial biomass, fungal biomass, yeast biomass, microalgae biomass, and/or combinations of any of the foregoing; wherein, preferably, said hydrolysates of microorganisms comprise or consist of fungal biomass, particularly yeast biomass.

In one embodiment, said fungal biomass comprises or consists of biomass of Aspergillus sp., Fusarium sp., Trichoderma sp., Ascobolus sp., Rhizopus sp., and/or combinations of any of the foregoing; wherein, preferably, said fungal biomass comprises or consists of biomass of Trichoderma reesei and/or Aspergillus niger.

In one embodiment, said yeast biomass comprises or consists of biomass of Saccharomyces sp., Yarrowia sp., Rhodosporidium sp., Cryptococcus sp., Trichosporon sp., Lipomyces sp., Rhodotorula sp., Candida sp., Cutaneotrichosporon sp., and/or combinations of any of the foregoing; wherein said yeast biomass preferably comprises or consists of biomass of Cutaneotrichosporon oleaginosus and/or Saccharomyces cerevisiae.

In one embodiment, said nitrogen source comprises or consists of any of organic nitrogen compounds, e.g. amines, amides, alkyl nitrates, nitrosamines, nitroarenes, and peroxyacyl nitrates; inorganic nitrogen compounds, e.g. ammonium salts, nitrate salts, and nitrite salts; amino acids; peptides; protein hydrolysates, e.g. peptone, tryptone, and other peptidic hydrolysates, wherein peptidic hydrolysates preferably comprise animal tissue, plant tissue, and/or components of said yeast; N-acetylglucosamine; and urea. In one embodiment, said nitrogen source comprises or consists of any of ammonium salts, amino acids, peptides, N-acetylglucosamine, and urea.

In one embodiment, said phosphate source comprises or consists of any of organic phosphate compounds, e.g. parathion, malathion, phospholipids, ATP, ADP, AMP, and organophosphate compounds; and inorganic phosphate salts, e.g. H₃PO₄, M₂HPO₄, MH₂PO₄, MHPO₄, and MPO₄, wherein M is a metal ion. For example, inorganic phosphate salts may be selected from, Na₃PO₄, Na₂HPO₄, NaH₂PO₄, K₃PO₄, K₂HPO₄, KH₂PO₄, calcium phosphate, ammonium dihydrogen phosphate and trisodium phosphate.

In one embodiment, the term "coproduct", as used herein, relates to a product of a yeast, e.g. a target compound, which is produced amongst at least one further product of said yeast e.g. a fatty acid. In one embodiment, said yeast produced microbial oil and further produces said at least one target compound. In one embodiment, when growing said yeast cell in step b), said yeast produces various products, including said target compound(s).

In one embodiment, the term "fermentative cultivation", as used herein, relates to a growing said yeast under fermentation conditions. For example, fermentation relates to cultivating a heterotrophic microorganism using a feedstock, such as aerobic and/or anaerobic cultivation. For example, fermentation conditions may comprise fermentation at a temperature in the range of from 10 °C to 45 °C, preferably in the range of from 15 °C to 40 °C, more preferably in the range of from 20 °C to 33 °C; at a pH in the range of from 4 to 9, preferably in the range of from 5 to 8, more preferably in the range of from 5.5 to 7.5; in a medium selected from minimal nitrogen media, minimal phosphate media, minimal sulfate media, and acetate rich media; and/or with dissolved oxygen (pO2) in a range of from 10 % to 90 %, preferably of from 20 % to 80 %, more preferably of from 40 % to 60 %. In one embodiment, step b) of growing said yeast comprises a fermentative cultivation.

In one embodiment, cultivating said at least one yeast cell comprises subjecting said at least one yeast cell to suitable growth conditions. In one embodiment, the terms "cultivating" and "growing" are used interchangeably. In one embodiment, growing said at least one yeast cell comprises contacting said at least one yeast cell with a biomass. In one embodiment, a biomass e.g. a biomass comprising animal tissue, plant tissue, and/or components of a yeast, and/or a hydrolysate of animal tissue, plant tissue, and/or components of a yeast, comprises a carbon source, e.g. polysaccharides and sugar, and/or comprises a nitrogen source, e.g. protein. In one embodiment, a method of the invention is performed in fed-batch manner, semi-continuous manner, or continuous mode-manner, preferably continuous mode-manner. In one embodiment, said step b) of growing said at least one yeast cell is performed in fed-batch manner, semi-continuous manner, or continuous mode-manner, preferably continuous mode-manner.

In one embodiment, said step b) of growing said at least one yeast cell in a medium comprises using a biomass as a substrate, such as using a biomass as the medium or using a biomass in the medium e.g. by supplementing said medium with biomass as a substrate. In one embodiment, said biomass used as a substrate for growing said yeast comprises a carbon source, a nitrogen source, and/or a phosphate source. In one embodiment, biomass is a substrate for said yeast. In one embodiment, the terms "biomass" and "substrate" are used interchangeably.

In one embodiment, said method further comprises a step of pretreating a substrate used for growing said at least one yeast cell in step b), e.g. a biomass comprising a carbon source, a nitrogen source, and/or a phosphate source, by
- mechanical pretreatment, preferably by milling, mixing, shredding, and/or sieving said substrate(s);
- dissolving said substrate(s) in a dissolvent, preferably in water;
- chemical hydrolysis of said substrate(s), preferably using an acid, e.g. sulfuric acid;
- thermal pretreatment of said substrate(s), preferably at a temperature of from 50 °C to 200 °C for 10 min to 240 min, more preferably at a temperature of from 80 °C to 170 °C for 30 min to 90 min;
- fermentative pretreatment of said substrate(s), preferably an anaerobic fermentation; and/or
- enzymatic pretreatment of said substrate(s) using one or more enzymes;
wherein said one or more enzymes preferably comprise proteases, e.g. endo- and exo-peptidases, serine endopeptidase, subtilisin A, and pepsin; and/or hydrolases, preferably glycoside hydrolases, more preferably glycoside hydrolases selected from α-amylases, amyloglucosidases, cellulases, hemicellulases, xylanases, xyloglucase, galactanase, arabinase, mannanase, lipase, glucoamylases, and pectinases.

Such pretreatment is sometimes herein referred to as "treatment o". In one embodiment, such step of pretreating said substrate, if present, is performed prior to step b) of growing said yeast in a medium, preferably in a medium comprising said pretreated substrate. In one embodiment, said fermentative pretreatment is an anaerobic fermentation using an anaerobic microorganism. In one embodiment, said fermentative pretreatment comprises a treatment of said substrate at a pH in the range of from pH 2.5 to pH 5.5, preferably pH 3 to pH 4, at a temperature in the range of from 20 °C to 50 °C, preferably 25 °C to 40 °C, at a p02 in the range of from 0-30%, preferably 5-10%, for 1-5 days, preferably 1-3 days, and/or at an agitation of from 50 rpm to 500 rpm.

In one embodiment, said pretreating said substrate is an enzymatic pretreatment which is a hydrolysis. In one embodiment, said step of pretreating said substrate comprises an enzymatic pretreatment which is a hydrolysis. A hydrolysis, e.g. of starch contained in biomass such as food residue biomass, may comprise two stages; in the first stage, α-amylase is used to liquefy the starch, thereby providing a solution including dextrins and glucose; in the second stage, the liquefied starch is subjected to glucoamylase. In one embodiment, said step of pretreating said substrate comprises an enzymatic pretreatment comprising a treatment with α-amylase and glucoamylase.

In one embodiment, the medium used to grow said yeast comprises a carbon source, nitrogen source, phosphate source, organic acid, trace metal, and/or vitamin. In one embodiment, said medium comprises any of carbohydrates; such as monosaccharides, preferably pentoses or hexoses, more preferably glucose, xylose, mannitol, arabinose; and oligosaccharides; protein hydrolysates such as oligo-amino acids and amino acids or other peptidic hydrolysates; fatty acids; organic acids, preferably acetic acid and/or a mix of volatile fatty acids; minerals; vitamins and trace elements, and combinations thereof. In one embodiment, the trace metal is selected from V, Mo, Cu, and Fe. In one embodiment, the vitamin is selected from vitamin C, vitamin B, vitamin A, and vitamin E.

In one embodiment, the carbon source comprises or consists of any carbon source selected from the group consisting of carbohydrates; monosaccharides, e.g. mannitol, pentoses, and hexoses, preferably glucose and xylose; oligosaccharides, e.g. maltose, lactose, sucrose, cellobiose, laminarose, cellodextrins, trehalose, raffinose, and melibiose; glycerol; amino acids; fatty acids; organic acid, e.g. acetic acid, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid, and the respective salt(s) of such acid; hydrolysates of animal tissues, plant tissues, industrial waste, food residues, or of microorganisms; and combinations of any of the foregoing; wherein, preferably, said carbon source comprises or consists of xylose, glucose, acetic acid and/or a volatile fatty acids mix. In one embodiment, the concentration of said carbon source in said medium is in a range from 50 to 400mM, preferably 200 to 300mM. Said glucose may be used alone or may e.g. be used in combination with suitable biomass hydrolysates, such as peptone, tryptone etc. In one embodiment, the hydrolysate is an algal hydrolysate, a lignocellulosic hydrolysate, a vegetable hydrolysate, a marine biomass hydrolysate, such as a hydrolysate of marine macro- and micro-algae, a corn hydrolysate, a wheat hydrolysate or other hydrolysate. In one embodiment, the term "volatile fatty acids mix" or "volatile fatty acids", relates to acetic acid, propionic acid and/or butyric acid, preferably to at least two of acetic acid, propionic acid and butyric acid, more preferably to acetic acid, propionic acid and butyric acid.

In one embodiment, said organic acid is selected from the group comprising acetic acids, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid and the respective salt(s) of such acid, as well as mixtures of any of the foregoing organic acids. Preferably, said organic acid is acetic acid or acetate. In one embodiment, said organic acid is acetic acid or acetate only; in another embodiment said organic acid is a combination of acetic acid or acetate, with any of the other aforementioned organic acids. It should be noted that the term "organic acid", as used herein is meant to encompass the respective organic acid irrespective of its degree of protonation, i.e. it is meant to encompass said acid in its protonated state(s) as well as deprotonated state(s), e.g. when in aqueous solution at a pH at which it is protonated or deprotonated, respectively, depending on the respective pKa-value(s). The term "organic acid", as used herein, is also meant to encompass salt(s) of the organic acid, e.g. the respective metal salts of such organic acid. Examples of such metal salts are the alkali salts or earth alkaline salts of the respective organic acid. The salts may be in their dissociated form or undissociated form. The term "mixtures of organic acids", as used herein is meant to encompass mixtures of organic acids in their respective acid form, i.e. with other organic acids, mixtures of organic acids in their acid form with other organic acids in their salt form, and mixtures of salts of organic acids with other salts of organic acids. It should also be noted that the term "organic acid", as used herein, does not encompass fatty acids or amino acids. The phrase "a carbon source and an organic acid", as used herein, implies that the "carbon source" is different from the "organic acid". Hence the two entities are chemically different. In one embodiment, the concentration of said organic acid is in a range of from 20 to 200 mM, preferably 30 to 100mM. In one embodiment, the medium is supplemented with an organic acid. Without wishing to be bound by any theory, the present inventors believe that the presence of organic acid, e.g. acetic acid/acetate allows to even further increase the lipid productivity, whereas the presence of a carbon source allows an increase in the total biomass.

In one embodiment, the capacity of the yeast to produce target compound(s) is further improved by genetic modification, e.g. by untargeted or targeted genetic alteration. In one embodiment, said untargeted genetic alteration comprises chemical- or radiation-induced mutation. In one embodiment, said radiation-induced mutation comprises any of UV, ionizing, gamma rays, X-rays, and neutron radiation. In one embodiment, said targeted genetic alteration comprises CRISPR-CAS systems, bacterial transformation, and /or plasmid-based insertion.

In one embodiment, the yeast comprises a genetic modification. In one embodiment, the yeast comprising a genetic modification is a genetically modified yeast. In one embodiment, said genetic modification improves the yield. In one embodiment, said genetic modification comprises transforming said yeast with at least one promoter and/or with at least target gene. For example, the yeast can be transformed with a promoter to increase the yield of the target compound(s). The target gene may be a gene involved in the synthesis of a target compound. Said yeast may be transformed with a target gene that allows said yeast to produce a target compound. In one embodiment, said target gene is selected from acetoaceteyl thiolase, HMG synthase, HMG reductase, squalene synthase, squalene epoxidase, lanosterol demethylase, sterol methyloxidase, sterol reductase, sterol dehydrogenase, sterol methyltransferase, sterol desaturase, sterol isomerase, 3-hydroxy-3-methylglutaryl coenzyme-a reductase, mevalonate kinase, farnesyl pyrophosphate synthase, phosphomevalonate kinase, geranylgeranyl pyrophosphate synthase, phytoene synthase and cyclase, phytoene desaturase, lycopene cyclase, pyridoxine phosphate oxidase, pyridoxal kinase, thiamin pyrophosphokinase, thiamine-phosphate kinase, TMP diphosphorylase, pantoate-beta-alanine ligase, dihydrofolate reductase, dihydrofolate synthase, and dihydro-6-hydroxymethylpterin pyrophosphokinase.

For example, enzymatic activity involved in the production of the target compound(s) can be increased by genetic modification, such as by increasing the copy number of an enzyme that is already present in a strain e.g. by adding one or more further genes; by increasing the expression levels (hyper-expression) of an enzyme that is already present e.g. by providing it with a promoter with higher activity; by adding a heterologous enzyme, particularly a gene that is not already present in a strain; by decreasing or preventing expression of a gene encoding an inhibitory function on the production of the target compound(s); and/or by modifying the sequence of an enzyme that is already present in a strain e.g. to enhance stability of the enzyme.

For example, enzymatic activity involved in the production of the target compound(s) can be decreased by genetic modification, such as by deleting a gene of an enzyme that is already present in a strain; by decreasing the expression levels of an enzyme that is already present e.g. by providing it with a promoter with lower activity; by increasing expression of a gene encoding an inhibitory function on the production of the target compound(s); by modifying the sequence of an enzyme that is already present in a strain e.g. to decrease stability of the enzyme.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) pretreating a substrate, e.g. biomass, to be used for growing said at least one yeast cell, such as a biomass comprising a carbon source, a nitrogen source, and/or a phosphate source;
iii) growing said at least one yeast cell in a medium comprising said pretreated substrate obtained in step ii), and thereby allowing said at least one yeast cell to produce at least one target compound;
iv) optionally, lysing said at least one yeast cell;
v) harvesting said at least one target compound;
vi) optionally, purifying said at least one target compound.

In one embodiment, said lysing said at least one yeast cell comprises or consists of any of enzymatic hydrolysis, temperature shock, chemical treatment, high-pressure homogenization, ultrasound homogenization, and any combination thereof. In a preferred embodiment, lysing said at least one yeast cell comprises or consists of an enzymatic hydrolysis. Such lysing is sometimes herein referred to as "treatment 1".

In one embodiment, said at least one target compound is comprised by a microbial oil produced by said yeast cell, and the microbial oil comprising said at least one target compound is obtained from said cell by lysing said cell. For example, the microbial oil can be intracellular microbial oil, and by lysing said cell, said microbial oil comprising said at least one target compound is obtained. Subsequently, the target compound(s) can be harvested from said microbial oil. Alternatively or additionally, the yeast may produce at least one target compound which is secreted, and such target compound(s) can be harvested without lysing said cell or prior to lysing said cell. In a case in which said yeast produces secreted target compound(s) and non-secreted target compound(s), said secreted target compound(s) may be harvested from said cell culture medium prior to or after lysing said cell, and said non-secreted target compound(s) may be harvested after lysing said cell. In a preferred embodiment, said target compound(s) are harvested from a microbial oil produced by said yeast after lysing said yeast.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) lysing said at least one yeast cell;
v) harvesting said at least one target compound;
vi) optionally, purifying said at least one target compound.

In one embodiment, the method of the invention further comprises a treatment step comprising any of water extraction, gravity-based separation, affinity-based separation, organic solvent extraction, ionic-liquid extraction, supercritical fluid extraction, liquid-liquid extraction, solid phase extraction, flash extraction, and any combination thereof. Such further treatment step is herein sometimes referred to as "treatment 2". In one embodiment, said harvesting comprises or follows said further treatment step comprising any of water extraction, gravity-based separation, affinity-based separation, organic solvent extraction, ionic-liquid extraction, supercritical fluid extraction, liquid-liquid extraction, solid phase extraction, flash extraction, and any combination thereof. For example, by performing such treatment 2, a product 1, as described herein, can be obtained. In one embodiment, a composition of the invention is obtained performing a method of the invention comprising said treatment step comprising any of water extraction, gravity-based separation, affinity-based separation, organic solvent extraction, ionic-liquid extraction, supercritical fluid extraction, liquid-liquid extraction, solid phase extraction, flash extraction, and any combination thereof.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) optionally, lysing said at least one yeast cell;
v) optionally, performing a gravity-based separation to obtain a hydrophilic fraction;
vi) performing a treatment step, e.g. of said hydrophilic fraction of step v) if present, comprising any of affinity-based separation, organic solvent extraction, ionic-liquid extraction, supercritical fluid extraction, liquid-liquid extraction, solid phase extraction, flash extraction, and any combination thereof;
vii) harvesting said at least one target compound;
viii) optionally, purifying said at least one target compound.

In one embodiment, the method of the invention further comprises a treatment step comprising any of water extraction, gravity-based separation, affinity-based separation, organic solvent extraction, ionic-liquid extraction, supercritical fluid extraction, liquid-liquid extraction, solid phase extraction, flash extraction, steam extraction, and any combination thereof. Such further treatment step is herein sometimes referred to as "treatment 3". In one embodiment, said harvesting comprises or follows said further treatment step comprising any of water extraction, gravity-based separation, affinity-based separation, organic solvent extraction, ionic-liquid extraction, supercritical fluid extraction, liquid-liquid extraction, solid phase extraction, flash extraction, steam extraction, and any combination thereof. For example, by performing such treatment 3, a product 3, as described herein, can be obtained.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) optionally, lysing said at least one yeast cell;
v) optionally, performing a gravity-based separation to obtain a solid fraction;
vi) preferably, performing a treatment step, e.g. of said solid fraction obtained in step v) if present, comprising any of water extraction, gravity-based separation, affinity-based separation, organic solvent extraction, ionic-liquid extraction, supercritical fluid extraction, liquid-liquid extraction, solid phase extraction, flash extraction, steam extraction, and any combination thereof;
vii) harvesting said at least one target compound;
viii) optionally, purifying said at least one target compound.

In one embodiment, the method of the invention further comprises a treatment step comprising any of degumming, neutralization, bleaching, distillation under inert gas, deodorization, and any combination thereof; preferably comprising any of neutralization, bleaching, deodorization, and any combination thereof; more preferably deodorization. Such further treatment step is herein sometimes referred to as "treatment 4". In one embodiment, said harvesting comprises or follows said further treatment step comprising any of degumming, neutralization, bleaching, distillation under inert gas, deodorization, and any combination thereof; preferably comprising any of neutralization, bleaching, deodorization, and any combination thereof; more preferably deodorization. For example, by performing such treatment 4, a product 5 (e.g. deodorized oil) or 6 (e.g. deodorant distillate), as described herein, can be obtained.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) optionally, lysing said at least one yeast cell;
v) preferably, performing a gravity-based separation to obtain a hydrophobic fraction;
vi) preferably, performing a treatment step, e.g. of said hydrophobic fraction of step v) if present, comprising any of degumming, neutralization, bleaching, distillation under inert gas, deodorization, and any combination thereof; preferably comprising any of neutralization, bleaching, deodorization, and any combination thereof; more preferably deodorization;
vii) harvesting said at least one target compound;
viii) optionally, purifying said at least one target compound.

In one embodiment, degumming, e.g. of a hydrophobic fraction, comprises any of water degumming, chemical degumming, physical degumming and/or enzymatic degumming. In one embodiment, said degumming is performed using a high-shear mixer in the range of from 200 to 10000 min⁻¹, preferably 300 to 5000 min⁻¹, more preferably 800 to 3000 min⁻¹ at a temperature in the range of from 4 to 150°C, preferably 60 to 120°C, more preferably 80 to 110°C. In one embodiment, said chemical degumming comprises degumming using phosphoric and/or citric acid, preferably at a concentration in the range of from 0.01 to 3.0% w/w, 0.05 to 1.0% w/w. In one embodiment, said chemical degumming comprises an EDTA treatment.

In one embodiment, said enzymatic degumming comprises degumming, e.g. a hydrophobic fraction, using phospholipase A2 (PLA2), phospholipases C and/or phospholipases D a concentration in the range of from 0.01-5.0% w/w, preferably 0.05-3.0% w/w. In one embodiment, said enzymatic degumming is performed at a pH value in the range of from pH 2 to pH 9, preferably, pH 3 to pH 7, more preferably pH 3.5 to pH 5.5. In one embodiment, said enzymatic degumming is performed at a temperature in the range of from 20-80°C, preferably 30-70°C, more preferably 40-60°C.

In one embodiment, said neutralization, e.g. of a hydrophobic fraction, is performed by alkaline addition, preferably addition of caustic soda and/or potassium hydroxide, e.g. at a concentration in the range of from 10 - 80% w/w, preferably 27 to 60%. In one embodiment, said neutralization is performed by adding said alkaline in such an amount that the free fatty acids and/or acid previously added are completely neutralized.

In one embodiment, said bleaching comprises treating, e.g. a hydrophobic fraction, with bleaching clay. In one embodiment, said bleaching is performed at a temperature in the range of from 60-150°C, preferably 70-120°C, more preferably 80-100°C. In one embodiment, said bleaching is performed at a bleaching clay concentration in the range of from 0.01-10 %w/w, preferably 0.1-7 %w/w, more preferably 0.5-5 %w/w. In one embodiment, said bleaching involves administering a stream of inert gas, such as N2, CO₂, and/or noble gases, preferably noble gases, more preferably argon. In one embodiment, said bleaching is performed involving administering and/or increasing a stream of inert gas, such as N2, CO₂, and/or noble gases, preferably noble gases, more preferably argon, to 0.5-15 ml of said inert gas per ml of a fraction, such as a hydrophobic fraction, per min (preferably 1-10 ml/ml per min, more preferably 1.5-5 ml/ml per min) during an adsorptive bleaching process. In one embodiment, said bleaching is performed for 1-800 minutes, preferably 10-300 min, more preferably 20-90 min. In one embodiment, said bleaching is followed by a cooling, e.g. of a bleached hydrophobic fraction, to a temperature in the range of from 20-100°C, preferably 30-90°C, more preferably 50-80°C, preferably to obtain a half-refined oil comprising said target compound(s).

In one embodiment, said deodorization comprises deodorization-deacidification and/or distillation e.g. short path distillation. In one embodiment, said deodorization-deacidification comprises pre-heating a fraction, e.g. a hydrophobic fraction, to a temperature in the range of from 50-150°C, preferably 70-120°C, more preferably 90-110°C. In one embodiment, said deodorization-deacidification comprises administering, e.g. to a pre-heated hydrophobic fraction, a stream of inert gas, such as N2, CO₂, and/or noble gases, preferably noble gases, more preferably argon. In one embodiment, said deodorization-deacidification comprises administering said stream of inert gas in an amount of 0.01 - 10.0 ml of said inert gas per ml of preheated hydrophobic fraction per min, preferably 0.1-5 ml/ml per min, more preferably 0.2-2 ml/ml per min; and/or administering said stream of inert gas in an amount of 0.5 - 15 ml of said inert gas per ml of hydrophobic fraction per min, preferably 1-10 ml/ml, more preferably 1.5-5 ml/ml. For example, a lower amount of inert gas, an amount of 0.01 - 10.0 ml of said inert gas per ml of preheated hydrophobic fraction per min, can be administered prior to administering a deodorization temperature, and a higher amount of inert gas, an amount of 0.5 - 15 ml of said inert gas per ml of hydrophobic fraction per min, can be administered during administering said deodorization temperature. In one embodiment, said deodorization comprises administering a deodorization temperature of 150-300 °C, preferably 180-270°C, more preferably 200-250°C. In one embodiment, during deodorization, the temperature is increased by about 10 °C each 30 to 60 minutes. In one embodiment, deodorization is performed for 1-1200 minutes, preferably 20-500 min, more preferably 30-180 min, preferably under a vacuum of about 0.1-10 hPa, preferably 0.5-8 hPa, more preferably 1-5 hPa.

In one embodiment, distillation e.g. short path distillation is performed at a temperature in the range of from 30-150°C, preferably 50-120°C, more preferably 65-100°C. In one embodiment, a condensate temperature is in the range of from 4-50°C, preferably 5-40°C, more preferably 10-30°C. In one embodiment, distillation e.g. short path distillation is performed at a pressure in the range of from 0.1-10 hPa, preferably 0.2-8 hPa, more preferably 0.5-4 hPa. In one embodiment, a feed hydrophobic fraction temperature subjected to distillation is in the range of from 4-50°C, preferably 5-40°C, more preferably 10-30°C. In one embodiment, distillation e.g. short path distillation is performed at a stirring rate in the range of from 50-1000 min⁻¹, preferably 100-800 min⁻¹, more preferably 200-500 min⁻¹.

In one embodiment, the method of the invention further comprises a treatment step comprising any of chromatography, affinity-based separation, organic solvent extraction, ionic-liquid extraction, supercritical fluid extraction, liquid-liquid extraction, solid phase extraction, flash extraction, steam extraction, vacuum distillation, distillation under inert or noble gases, and any combination thereof. Such further treatment step is herein sometimes referred to as "treatment 5". In one embodiment, said harvesting of step d) comprises or follows said further treatment step comprising any of chromatography, affinity-based separation, organic solvent extraction, ionic-liquid extraction, supercritical fluid extraction, liquid-liquid extraction, solid phase extraction, flash extraction, steam extraction, vacuum distillation, distillation under inert or noble gases, and any combination thereof. For example, by performing such treatment 5, a product 7 (e.g. target compound having a purity of at least 90%), as described herein, can be obtained. In one embodiment, such treatment 5 comprises a purification of said target compound(s).

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) optionally, lysing said at least one yeast cell;
v) performing a gravity-based separation to obtain a hydrophobic fraction;
vi) optionally, performing a treatment step of said hydrophobic fraction obtained in step v), comprising any of degumming, neutralization, bleaching, distillation under inert gas, deodorization, and any combination thereof; preferably comprising any of neutralization, bleaching, deodorization, and any combination thereof; more preferably deodorization;
vii) performing a treatment step of said hydrophobic fraction obtained in step v) or of said treated fraction obtained in step vi) if present, comprising any of chromatography, affinity-based separation, organic solvent extraction, ionic-liquid extraction, supercritical fluid extraction, liquid-liquid extraction, solid phase extraction, flash extraction, steam extraction, vacuum distillation, distillation under inert or noble gases, and any combination thereof;
viii) harvesting said at least one target compound;
ix) optionally, purifying said at least one target compound.

In one embodiment, said harvesting in step d) comprises density-based separation. In its simplest form, such density-based separation method may be a process wherein the culture of the yeast is simply allowed to stand for a period of time as a result of which the lipid phase will separate from an aqueous phase due to different densities and/or solubility in water. This may be combined with a subsequent decantation, aspiration or other mechanical removal of the lipid-phase from the culture. In other embodiments, separation may occur by way of a gravity-assisted phase separation, by way of a centrifugation, alone or in combination with a subsequent mechanical removal or the lipid-phase, e. g. a decantation or aspiration.

In one embodiment, the method of the invention comprises a step of separating said target compound(s) from said yeast cell(s), said medium, and/or other products of said yeast, to obtain said at least one target compound. In one embodiment, said step of separating is a step of harvesting said at least one target compound. In one embodiment, the terms "harvesting" and "separating" are used interchangeably. In one embodiment, said harvesting in step d) comprises density-based separation, drying, floating, solvent-based extraction, chromatography, distillation, maceration, supercritical fluid extraction, enfleurage, press extraction, demulsification, decantation, and/or aspiration, preferably density-based separation. In one embodiment, said density-based separation is selected from separation based on natural gravity, gravity-assisted phase separation, and centrifugation, wherein each of said separation based on natural gravity, gravity-assisted phase separation, and centrifugation, is performed alone or in combination with a decantation, aspiration or other mechanical harvesting method. In one embodiment, the step d) of harvesting said target compound(s) is a step of obtaining said target compound(s). In one embodiment, said harvesting in step d) comprises harvesting said at least one target compound from said medium, from a produced yeast oil, e.g. from an unsaponifiable matter of a produced yeast oil, and/or from a cell debris, preferably from an unsaponifiable matter of a produced yeast oil, optionally after a step of oil extraction. In one embodiment, said harvesting in step d) further comprises harvesting a compound other than the at least one target compound, preferably an oil, sugar, amino acid, and/or organic acid. In one embodiment, said step d) of harvesting said at least one target compound is a step of obtaining said at least one target compound, and optionally obtaining a product of said yeast other than the at least one target compound, preferably an oil, sugar, amino acid, and/or organic acid. For example, the microbial oil comprising target compound(s) can be obtained by a method such as density-based separation, and said target compound(s) can be harvested from said microbial oil; furthermore, other products e.g. compounds can be harvested from the microbial oil, such as fatty acids, and/or from the aqueous phase, particularly the cell culture medium, such as sugars and amino acids.

In one embodiment, said purifying, e.g. said purifying of any of the embodiments herein, is performed using a separation method comprising chromatography, affinity-based separation, organic solvent extraction, ionic-liquid extraction, supercritical fluid extraction, liquid-liquid extraction, solid phase extraction, flash extraction, steam extraction, vacuum distillation, distillation under inactive or noble gases, and/or deodorization. In one embodiment, said purifying, e.g. in step e), comprises solid phase extraction. In one embodiment, said purifying comprises methylation. For example, methylation can be used to separate triglycerides (TAGs) and free fatty acids (FFAs) from target compounds such as squalene and ergosterols. In one embodiment, the at least one target compound is separated from a hydrophobic fraction using solid phase extraction, distillation and/or methylation. In one embodiment, obtaining said at least one target compound comprises harvesting said at least one target compound, and optionally purifying said at least one target compound.

In one embodiment, harvesting said at least one target compound and/or purifying said at least one target compound comprises bleaching a microbial oil comprising said at least one target compound to obtain a bleached oil comprising said target compound(s). In one embodiment, bleaching is performed at a temperature in the range of from 90 °C to 125 °C, for 15 to 45 min, preferably 20 to 30 min, at a pressure of from 6000 Pa to 17000 Pa, and/or by adding bleaching clay at a concentration of 0.5-5% w/w of oil.

For example, the bleaching of oils such as edible oils and fats is used to refine crude oils and fats, which removes contaminants that adversely impact the appearance and performance of these triglyceride (triacylglycerol)-based materials. The bleaching of the oils and fats effectively removes some of the color, reduces the contents of chlorophyll, residual soap and gums, trace metals, and oxidation products. In one embodiment, the method of the invention comprises a step of refining. In one embodiment, refining comprises bleaching, deodorizing and/or neutralization.

In one embodiment, harvesting said at least one target compound and/or purifying said at least one target compound comprises refining a microbial oil comprising said at least one target compound to obtain a refined oil comprising said target compound(s), preferably by degumming, bleaching, and deodorizing said microbial oil, wherein said deodorizing is performed at a temperature of about 210°C. In one embodiment, harvesting said at least one target compound and/or purifying said at least one target compound comprises refining a microbial oil comprising said at least one target compound to obtain a refined oil comprising said target compound(s), preferably by degumming, bleaching, and deodorizing said microbial oil, wherein said deodorizing is performed at a temperature of about 230°C. In one embodiment, when referring to the term "refined I", said refining comprising said deodorizing at a temperature of about 210°C is meant. In one embodiment, when referring to the term "refined II", said refining comprising said deodorizing at a temperature of about 230°C is meant. In one embodiment, said method of the invention comprises a step of deodorizing at a temperature in the range of from 200° to 240°C, preferably of about 210°C to about 230°C.

In one embodiment, the method for producing the at least one target compound comprises the steps:
i) providing at least one yeast cell, wherein said yeast cell is a yeast from the family Trichosporonaceae; wherein, preferably, said yeast is from the genus *Cutaneotrichosporon;*
ii) optionally, pretreating a substrate, e.g. biomass, to be used for growing said at least one yeast cell, such as a biomass comprising a carbon source, a nitrogen source, and/or a phosphate source;
iii) growing said at least one yeast cell in a medium comprising a carbon source, a nitrogen source, and a phosphate source, e.g. a pretreated substrate obtained in step ii) if present, and thereby allowing said at least one yeast cell to produce at least one target compound;
iv) optionally, lysing said at least one yeast cell;
v) optionally, performing a gravity-based separation to obtain whole cells or whole cell components;
vi) performing a treatment step, e.g. of said whole cells or whole cell components of step v) if present, comprising any of floating, coagulation, centrifugation, homogenization, drying, granulation, and any combination thereof;
vii) harvesting said at least one target compound;
viii) optionally, purifying said at least one target compound.

In one embodiment, the method of the invention further comprises a treatment step comprising any of gravity-based separation, floating, coagulation, centrifugation, homogenization, drying, granulation, and any combination thereof. Such further treatment step is herein sometimes referred to as "treatment 8". In one embodiment, said harvesting comprises or follows said further treatment step comprising any of gravity-based separation, floating, coagulation, centrifugation, homogenization, drying, granulation, and any combination thereof. For example, by performing such treatment 8, a product 8, as described herein, can be obtained.

The term "single cell oil" or "SCO", as used herein, relates to lipids produced by an oleaginous microorganism, e.g. yeast oil. In one embodiment, the term "microbial lipid" is used interchangeably with "single cell oil" or "microbial oil". Typically, microbial lipids are rich in unsaturated fatty acids. Particularly, single cell oil is an oil obtainable from single-celled microorganisms such as yeasts. In one embodiment, the term "unsaponifiable matter" or "unsaponification matter", as used herein, relates to components of a fatty substance that fail to form soaps when treated with alkali and remain insoluble in water but soluble in organic solvents.

In one embodiment, the composition of the invention is advantageous in that it comprises high amounts of terpenoids such as ergosterol and/or ergosterol derivatives. In one embodiment, the composition of the invention is a composition obtained by a method of the invention. In one embodiment, the composition of the invention is a composition obtainable by a method of the invention. In one embodiment, the composition of the invention is a fraction, such as a fraction obtained performing density-based separation, solvent-based extraction, chromatography, distillation, maceration, supercritical fluid extraction, enfleurage, press extraction, demulsification, decantation, and/or aspiration, preferably density-based separation. In one embodiment, the composition of the invention is a fraction, such as a fraction obtained lysing said yeast cells and performing density-based separation, solvent-based extraction, chromatography, distillation, maceration, supercritical fluid extraction, enfleurage, press extraction, demulsification, decantation, and/or aspiration, preferably density-based separation with said lysed cells. In one embodiment, in the composition of the invention, said at least one target compound has a purity of at least 1 %, 10%, 20%, 30 %, 40 %, or 50 %, preferably of at least 60 %, 70 %, or 80 %, more preferably of at least 90 %, even more preferably of at least about 95%. In one embodiment, the composition of the invention comprises the target compound, e.g. ergosterol and/or squalene, with a purity of at least about 90%, preferably of at least about 95%. In one embodiment, said at least one target compound is present in said composition in a concentration of 0.1% to 50 % by weight. In a preferred embodiment, the composition of the invention comprises squalene in a concentration of at least 0.2 % w/w, e.g. at least 0.5 % w/w. In a preferred embodiment, the composition of the invention comprises ergosterol in a concentration of at least 0.5 % w/w. In a preferred embodiment, the composition of the invention comprises ergosterol in a concentration of at least 0.5 % w/w. In a preferred embodiment, the composition of the invention comprises α-tocopherol in a concentration of at least 0.001 % w/w. In a preferred embodiment, the composition of the invention comprises α-tocotrienol in a concentration of at least 0.0005 % w/w. In a preferred embodiment, the composition of the invention comprises tocochromanols in a concentration of at least 0.0015% w/w.

In one embodiment, the composition of the invention comprises triglycerides in the range of from 50-95%w/w, and further comprises a phenolic compound in the range of from 0.1-10% w/w, a vitamin in the range of from 0.05 to 2 % w/w, and/or a terpenoid in the range of from 1-50% w/w. In one embodiment, the composition of the invention comprises water and vitamin B₁, vitamin B₂, vitamin B₃, vitamin B₅, vitamin B₆, and/or vitamin B₁₂, said vitamin(s) in the range of from 0.00001-5% w/w, and further comprises sugar in the range of from 1-10% w/w, amino acids in the range of from 1 to 5 % w/w, and/or peptides/proteins in the range of from 0.5-8%. In one embodiment, the composition of the invention comprises an aqueous phase and a lipid phase; wherein said aqueous phase comprises water and vitamin B₁, vitamin B₂, vitamin B₃, vitamin B₅, vitamin B₆, and/or vitamin B₁₂, said vitamin(s) in the range of from 0.00001-5% w/w, and further comprises sugar in the range of from 1-10% w/w, amino acids in the range of from 1 to 5 % w/w, and/or peptides/proteins in the range of from 0.5-8%; and wherein said lipid phase comprises triglycerides in the range of from 50-95%w/w, and further comprises a phenolic compound in the range of from 0.1-10% w/w, a vitamin in the range of from 0.05 to 2 % w/w, and/or a terpenoid in the range of from 1-50% w/w.

In one embodiment, the composition of the invention comprises solid biomass in the range of from 30-95 % w/w, preferably in the range of from 50 - 92 % w/w; acylglycerols in the range of from 3 - 40% w/w, preferably in the range of from 10 - 30 % w/w; squalene in the range of from 0.0 - 35% w/w, preferably in the range of from 0.1 - 20% w/w; ergosterol in the range of from 0.0 - 40% w/w, preferably in the range of from 0.5 - 20 % w/w; free fatty acids in the range of from 0.0 - 4% w/w, preferably in the range of from 0.1 - 0.5% w/w; phospholipids, sphingolipids, and/or ceramides in the range of from 0.0 - 20% w/w, preferably in the range of from 0.5 - 10% w/w; β-carotenes in the range of from 0.0-5% w/w, preferably in the range of from 0.2 - 3% w/w; and tocopherol and/or tocotrienols in the range of from 0.0-4% w/w, preferably in the range of from 0.1 - 3% w/w. In some embodiments, said composition is referred to as "product 2".

In one embodiment, the composition of the invention comprises solid biomass in the range of from 30-95 % w/w, preferably in the range of from 50 - 92 % w/w; acylglycerols in the range of from 3 - 40% w/w, preferably in the range of from 10 - 30 % w/w; terpenoid(s) in the range of from 0.0 - 60% w/w, preferably in the range of from 3.0 - 50% w/w; phenolic compound(s) in the range of from 0.0 - 10% w/w, preferably in the range of from 0.5 - 5% w/w; free fatty acids in the range of from 0.0 - 4% w/w, preferably in the range of from 0.1 - 0.5% w/w; phospholipids, sphingolipids, and/or ceramides in the range of from 0.0 - 20% w/w, preferably in the range of from 0.5 - 10% w/w. In some embodiments, said composition is referred to as "product 2".

In one embodiment, the composition of the invention comprises solid biomass in the range of from 0-10 % w/w, preferably in the range of from 0 - 1 % w/w; acylglycerols in the range of from 10 - 60% w/w, preferably in the range of from 20 - 40% w/w; squalene in the range of from 0.0 - 45% w/w, preferably in the range of from 0.1 - 35% w/w; ergosterol in the range of from 0.0 - 60% w/w, preferably in the range of from 0.5 - 40% w/w; free fatty acids in the range of from 0.0 - 6% w/w, preferably in the range of from 0.1 - 0.5% w/w; phospholipids, sphingolipids, and/or ceramides in the range of from 0.0 - 30% w/w, preferably in the range of from 0.5 - 20% w/w; β-carotenes in the range of from 0.0-10% w/w, preferably in the range of from 0.2 - 8% w/w; and tocopherol and/or tocotrienols in the range of from 0.0-4% w/w, preferably in the range of from 0.1 - 3% w/w. In some embodiments, said composition is referred to as "product 3".

In one embodiment, the composition of the invention comprises solid biomass in the range of from 0- 10% w/w, preferably in the range of from 0 - 1% w/w; acylglycerols in the range of from 10 - 60% w/w, preferably in the range of from 20 - 40% w/w; terpenoid(s) in the range of from 0.0 - 80% w/w, preferably in the range of from 5.0 - 60% w/w; phenolic compound(s) in the range of from 0.0 - 15% w/w, preferably in the range of from 0.5 - 10% w/w; free fatty acids in the range of from 0.0 - 6% w/w, preferably in the range of from 0.1 - 0.5% w/w; phospholipids, sphingolipids, and/or ceramides in the range of from 0.0 - 30% w/w, preferably in the range of from 0.5 - 20% w/w. In some embodiments, said composition is referred to as "product 3". In one embodiment, the term "solid biomass", as used herein, relates to cell debris, preferably cell debris without the target compounds. In one embodiment, particularly in the context of weight ranges of a composition of the invention, solid biomass, e.g. cell debris, does not include target compound(s).

In one embodiment, the composition of the invention comprises acylglycerols in the range of from 60 - 98% w/w, preferably in the range of from 80 - 92% w/w; squalene in the range of from 0.0 - 35% w/w, preferably in the range of from 0.1 - 20% w/w; ergosterol in the range of from 0.0 - 40% w/w, preferably in the range of from 0.5 - 20% w/w; free fatty acids in the range of from 0.0 - 4% w/w, preferably in the range of from 0.1 - 0.5% w/w; phospholipids, sphingolipids, and/or ceramides in the range of from 0.0 - 0.5% w/w, preferably in the range of from 0.01 - 0.2% w/w; β-carotenes in the range of from 0.0-15% w/w, preferably in the range of from 0.2 - 10% w/w; and tocopherol and/or tocotrienols in the range of from 0.0-10% w/w, preferably in the range of from 0.1 - 5% w/w. In some embodiments, said composition is referred to as "product 4".

In one embodiment, the composition of the invention comprises acylglycerols in the range of from 60 - 98% w/w, preferably in the range of from 80 - 92% w/w; terpenoid(s) in the range of from 0.0 - 60% w/w, preferably in the range of from 3.0 - 50% w/w; phenolic compound(s) in the range of from 0.0 - 10% w/w, preferably in the range of from 0.5 - 5% w/w; free fatty acids in the range of from 0.0 - 4% w/w, preferably in the range of from 0.1 - 0.5% w/w; phospholipids, sphingolipids, and/or ceramides in the range of from 0.0 - 3% w/w, preferably in the range of from 0.01 - 1%w/w. In some embodiments, said composition is referred to as "product 4".

In one embodiment, the composition of the invention comprises acylglycerols in the range of from 95 - 98% w/w; terpenoid(s) in the range of from 0.7 - 1.3% w/w; phenolic compound(s) in the range of from 0.005 - 0.015% w/w; free fatty acids in the range of from 0.75 - 3% w/w; phospholipids, sphingolipids, and/or ceramides in the range of from 0.025 - 0.75% w/w.

In one embodiment, the composition of the invention comprises acylglycerols in the range of from 60 - 98% w/w, preferably in the range of from 80 - 92% w/w; squalene in the range of from 0.0 - 30% w/w, preferably in the range of from 0.1 - 10% w/w; ergosterol in the range of from 0.0 - 35% w/w, preferably in the range of from 0.5 - 25% w/w; free fatty acids in the range of from 0.0 - 4% w/w, preferably in the range of from 0.01 - 0.05% w/w; phospholipids, sphingolipids, and/or ceramides in the range of from 0.0 - 0.5% w/w, preferably in the range of from 0.01 - 0.02% w/w; β-carotenes in the range of from 0.0-10% w/w, preferably in the range of from 0.2 - 5% w/w; and tocopherol and/or tocotrienols in the range of from 0.0-8% w/w, preferably in the range of from 0.01 - 4% w/w. In some embodiments, said composition is referred to as "product 5".

In one embodiment, the composition of the invention comprises acylglycerols in the range of from 60 - 98% w/w, preferably in the range of from 80 - 92% w/w; terpenoid(s) in the range of from 0.0 - 50% w/w, preferably in the range of from 3.0 - 40% w/w; phenolic compound(s) in the range of from 0.0 - 10% w/w, preferably in the range of from 0.5 - 5% w/w; free fatty acids in the range of from 0.0 - 2% w/w, preferably in the range of from 0.01 - 0.05% w/w; phospholipids, sphingolipids, and/or ceramides in the range of from 0.0 - 0.5% w/w, preferably in the range of from 0.01 - 0.02% w/w. In some embodiments, said composition is referred to as "product 5".

In one embodiment, the composition of the invention comprises acylglycerols in the range of from 6 - 8% w/w; terpenoid(s) in the range of from 65 - 75% w/w; phenolic compound(s) in the range of from 0.2 - 0.6% w/w; free fatty acids in the range of from 16 - 20% w/w; phospholipids, sphingolipids, and/or ceramides in the range of from 0.05 - 0.15% w/w.

In one embodiment, the composition of the invention comprises acylglycerols in the range of from 1 - 30% w/w, preferably in the range of from 5-15% w/w; squalene in the range of from 0.05-50% w/w, preferably in the range of from 10 - 40% w/w; ergosterol in the range of from 0.07-65% w/w, preferably in the range of from 0.5 - 50% w/w; free fatty acids in the range of from 2-20% w/w, preferably in the range of from 5-15% w/w; phospholipids, sphingolipids, and/or ceramides in the range of from 0.01 - 0.5% w/w, preferably in the range of from 0.05 - 0.1% w/w; β-carotenes in the range of from 0.01-15% w/w, preferably in the range of from 0.05-10% w/w; and tocopherol and/or tocotrienols in the range of from 0.01-10% w/w, preferably in the range of from 0.05-5% w/w. In some embodiments, said composition is referred to as "product 6".

In one embodiment, the composition of the invention comprises acylglycerols in the range of from 1 - 30% w/w, preferably in the range of from 5-15% w/w; terpenoid(s) in the range of from 0.0 - 80% w/w, preferably in the range of from 10 - 65% w/w; phenolic compound(s) in the range of from 0.0 - 25% w/w, preferably in the range of from 0.05 - 15% w/w; free fatty acids in the range of from 2-20% w/w, preferably in the range of from 5-15% w/w; phospholipids, sphingolipids, and/or ceramides in the range of from 0.01 - 0.5% w/w, preferably in the range of from 0.05 - 0.1% w/w. In some embodiments, said composition is referred to as "product 6".

In one embodiment, the method of the invention comprises obtaining a composition as defined herein. In one embodiment, the method of the invention comprises producing a composition as defined herein.

The terms "of the [present] invention", "in accordance with the invention", "according to the invention" and the like, as used herein are intended to refer to all aspects and embodiments of the invention described and/or claimed herein. In one embodiment, the terms "at least one" and "one or more" are used interchangeably.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

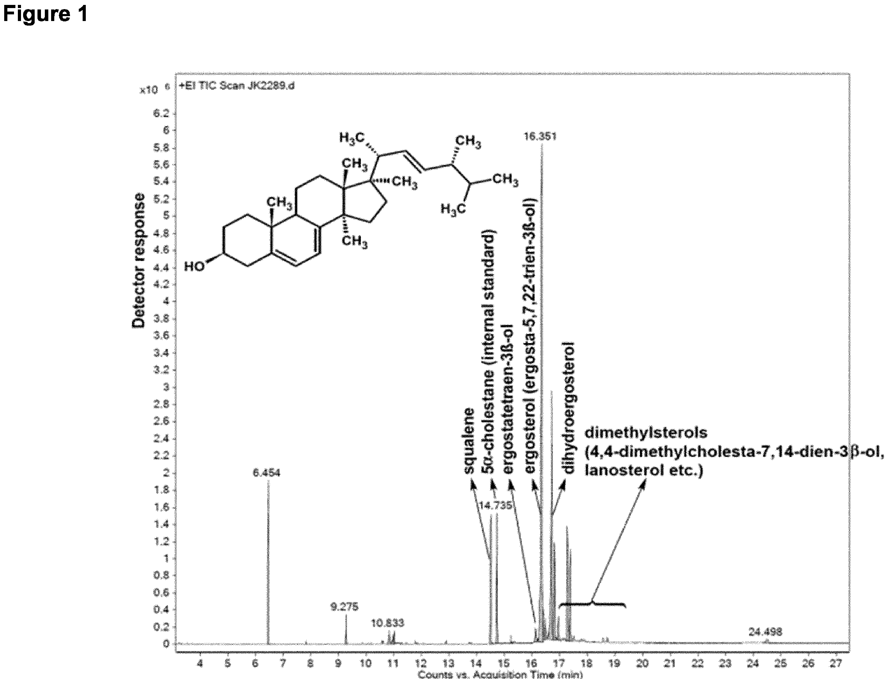

**Figure 1** shows a squalene analysis using GC-MS of unsaponification matter from yeast oil.

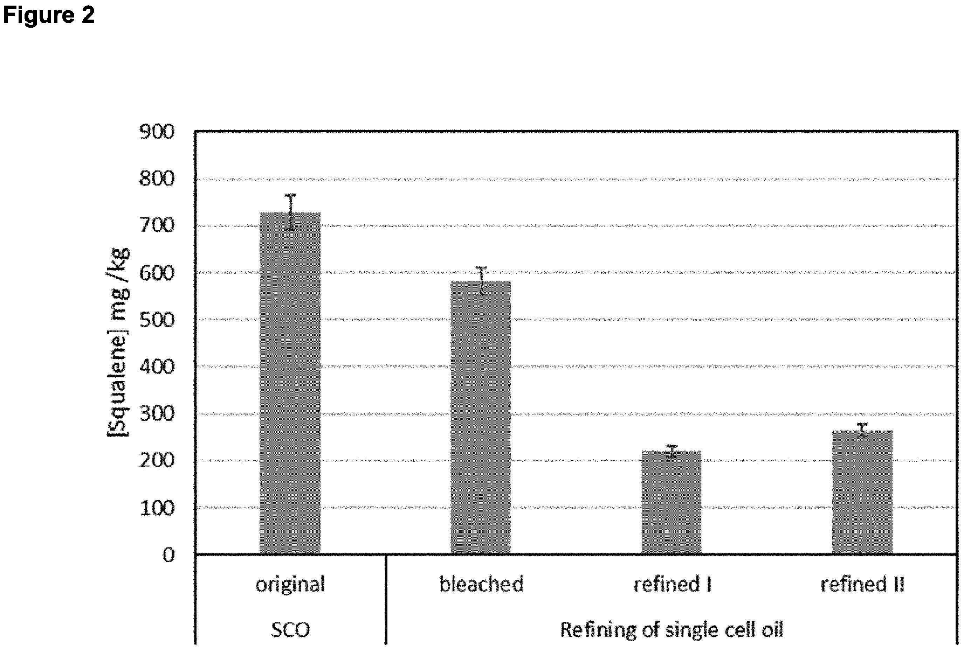

**Figure 2** shows the squalene concentration of unsaponification matter from yeast oil; crude oil and refined oil. Particularly, the squalene concentration of unsaponification matter from the hydrophobic fraction (product 4 and product 5) is shown. For product 4 (original sco), and product 5 when X= 90°C (bleached), 110°C (Refined I), 120°C (Refined II), and Y= 210°C.

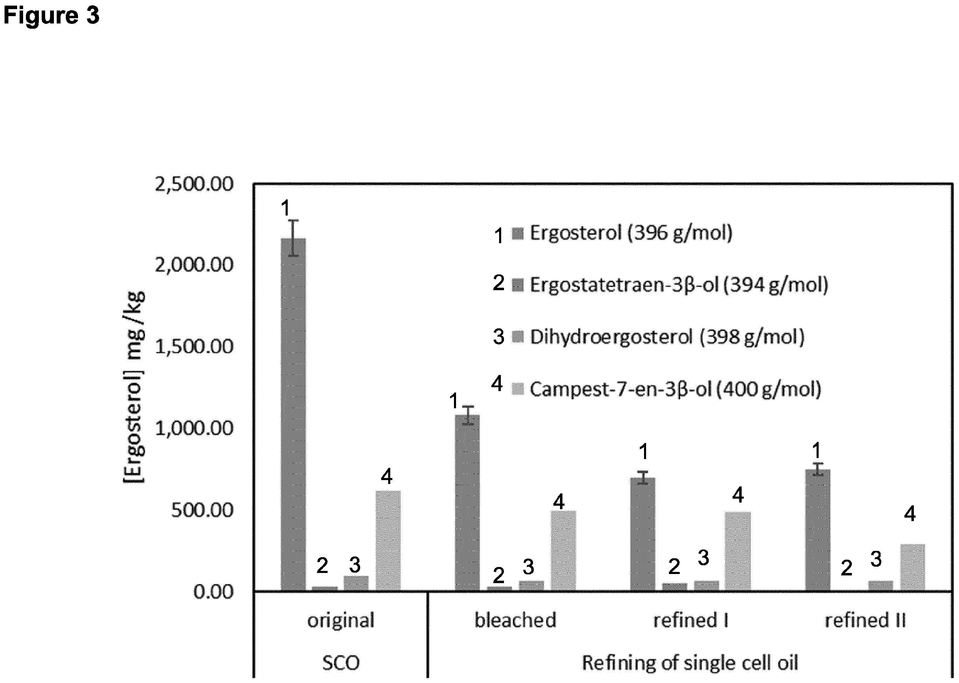

**Figure 3** shows the ergosterol concentration of unsaponification matter from yeast oil; crude and refined oil. Particularly, the ergosterols concentration of unsaponification matter from the hydrophobic fraction (product 4 and product 5) is shown. Ergostatetraen and campest-7-en are ergosterol derivatives. For product 4 (original sco), and product 5 when X= 90°C (bleached), 110°C (Refined I), 120°C (Refined II), and Y= 210°C. The inventors observed that increasing the Y results in a reduction of the content of target compound (e.g. ergosterol concentration) in product 5.

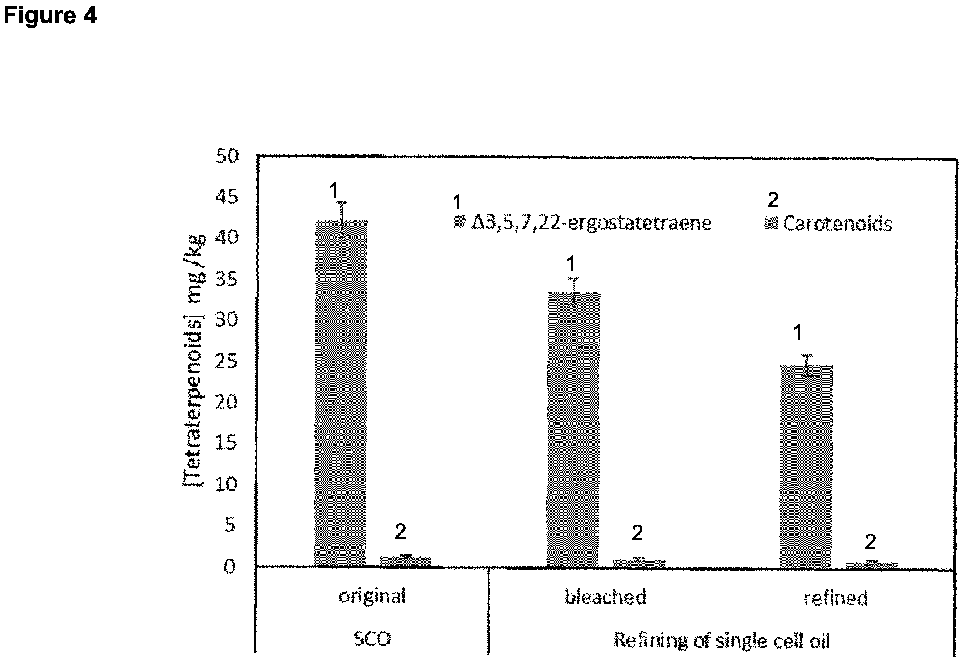

**Figure 4** shows the carotenoid and ergostatetraene concentration of unsaponification matter from yeast oil; crude and refined oil. Particularly, the ergosterols concentration of unsaponification matter from the hydrophobic fraction (product 4 and product 5) is shown. For product 4 (original sco), and product 5 when X= 90°C (bleached), 110°C (Refined), and Y= 210°C. The inventors observed that increasing the X results in a reduction of the content of target compound (e.g. carotenoid and ergostatetraene concentration) in product 5.

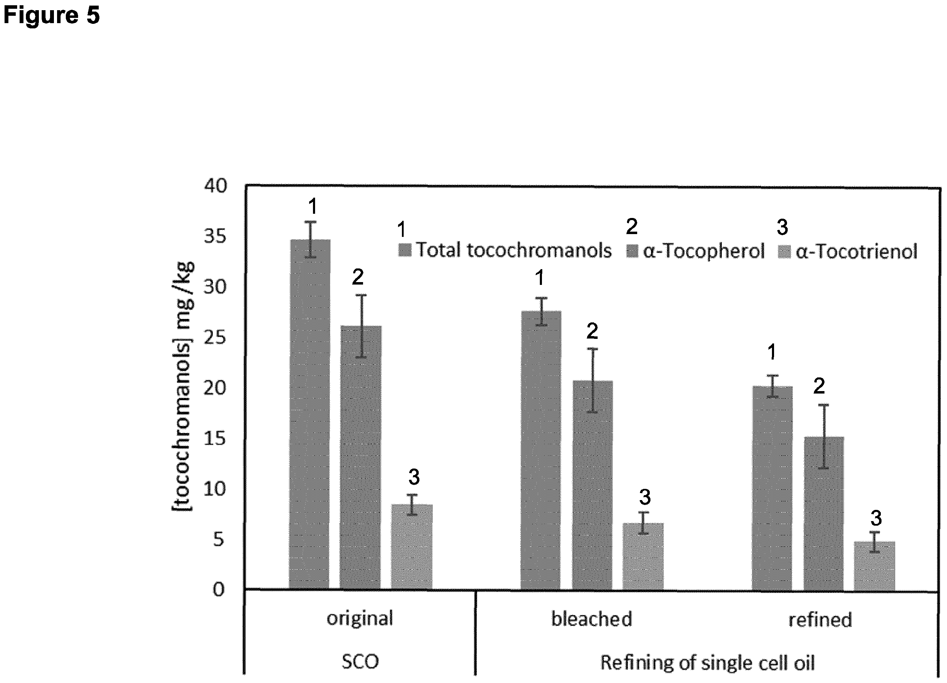

**Figure 5** shows the phenolic compound concentrations, e.g. tocochromanols such as α-tocopherol and α-tocotrienol, of unsaponification matter from yeast oil; crude and refined oil.

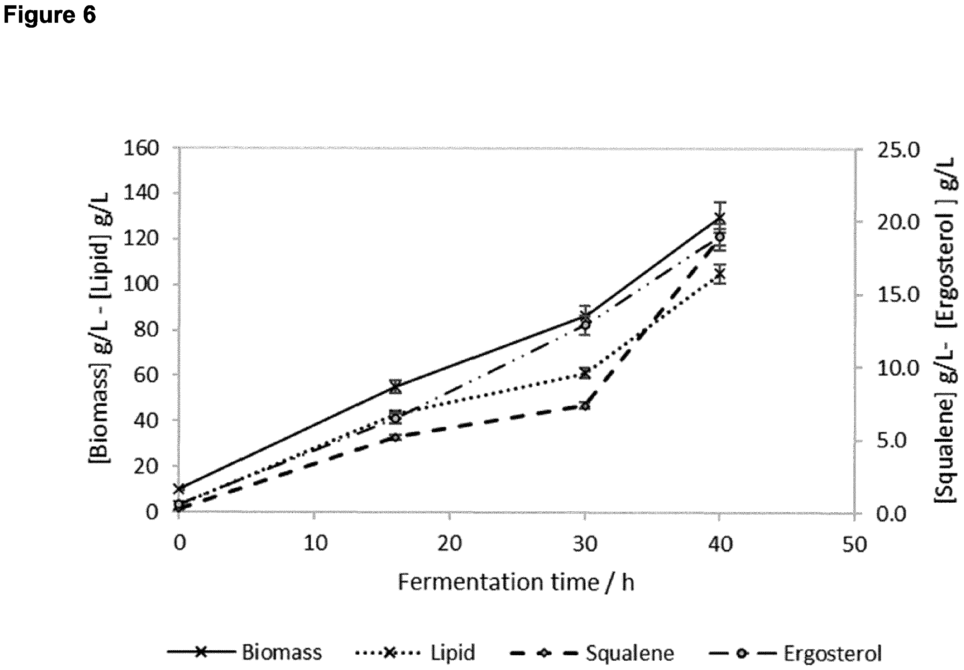

**Figure 6** shows the increase of yeast biomass, lipid, squalene and ergosterol concentration over fermentation time. Fermentation was done at scale of 3 L, temp.: 28°C, pH: 6.5, DO: 25-60, stirrer: 200 -800, and aeration: 0.5 -1.5 vvm. As shown, the method of the invention is highly effective in producing microbial lipids and terpenoids including squalene and ergosterol.

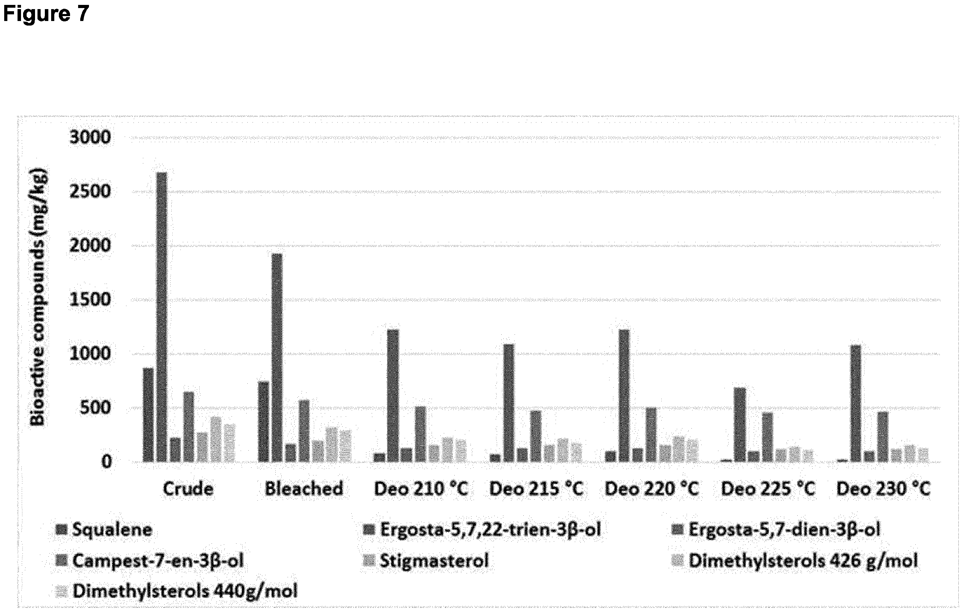

**Figure 7** shows squalene and ergosterol/ergosterol derivatives in product 4 (crude) and product 5 when (X= 90°C and Y= 210°C, 215°C, 220°C, 225°C and 230°C). As shown, the various embodiments of the method of the invention allowed to obtain said at least one target compound, particularly a product comprising at least one target compound. Particularly, the inventors observed that increasing the Y results in a reduction of the content of target compound in product 5.

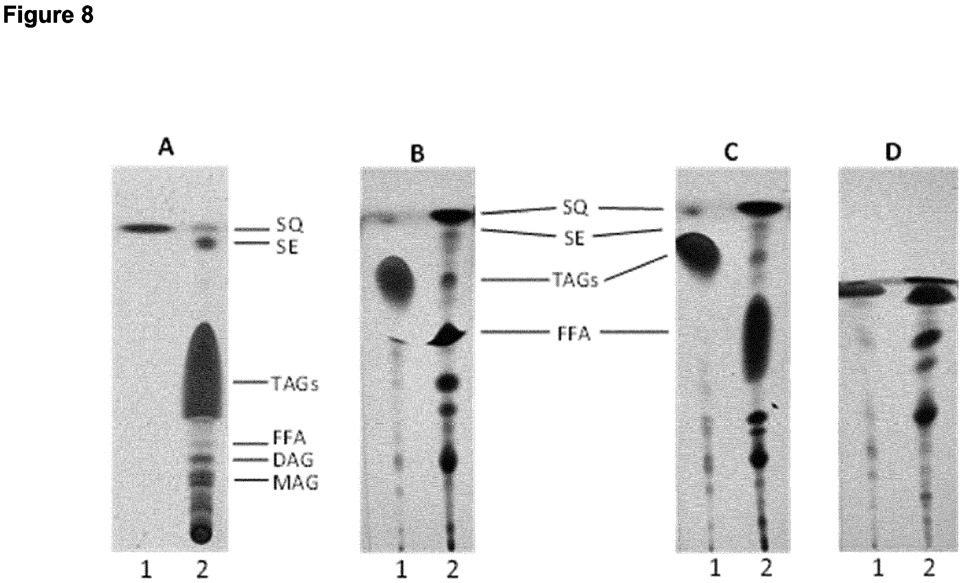

**Figure 8** shows thin-layer chromatography (TLC) of oil in different mobile phases; A) Lipid separation of squalene standard (line 1) and Product 5 (line 2) with n-hexane: diethyl ether: acetic acid (85:15:1, v/v/v) as observed by [1]; B) hexane: diethyl ether: propionic acid (80:20:2, v/v/v); C) hexane: diethyl ether: acetic acid (80:20:2, v/v/v); D) hexane: diethyl ether: formic acid (80:20:2, v/v/v) with Product 4 (line 1) and Product 6 (line 2); (SQ = squalene, SE = steryl ester, TAGs = triglycerides, FFA = free fatty acids, DAG = diacylglycerol, MAG = monoacylglycerol). As shown, the method of the invention allows to efficiently produce terpenoids such as squalene. The figure demonstrates the fractionation and purification of ergosterol and squalene by using chromatography methods with different mobile phases.

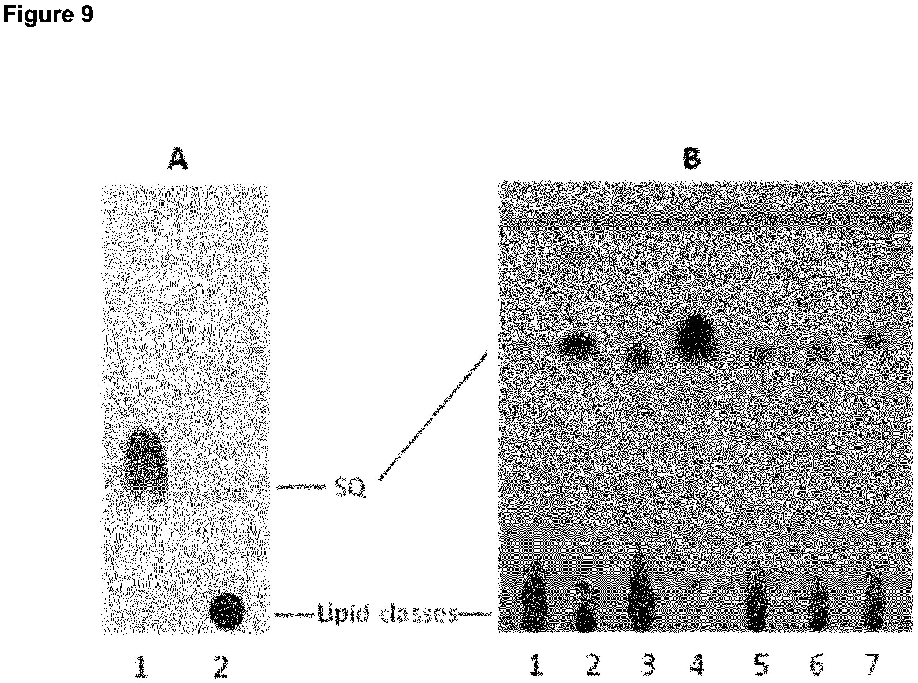

**Figure 9** shows TLC for separation of squalene; A) Lipid separation of squalene standard (line 1) and Product 5 (line 2) with n-hexane, B) separation by hexane: chloroform (9:1, v/v) of Product 5 (line 1), Product 6 (line 2), olive oil (line 3), squalene standard (line 4) and three fermentation samples taken after 116 h (line 5), 146 h (line 6) and 211 h (line 7) after inoculation; SQ = squalene, Lipid classes = other components of the lipid samples. As shown, the method of the invention allows to efficiently produce terpenoids such as squalene.

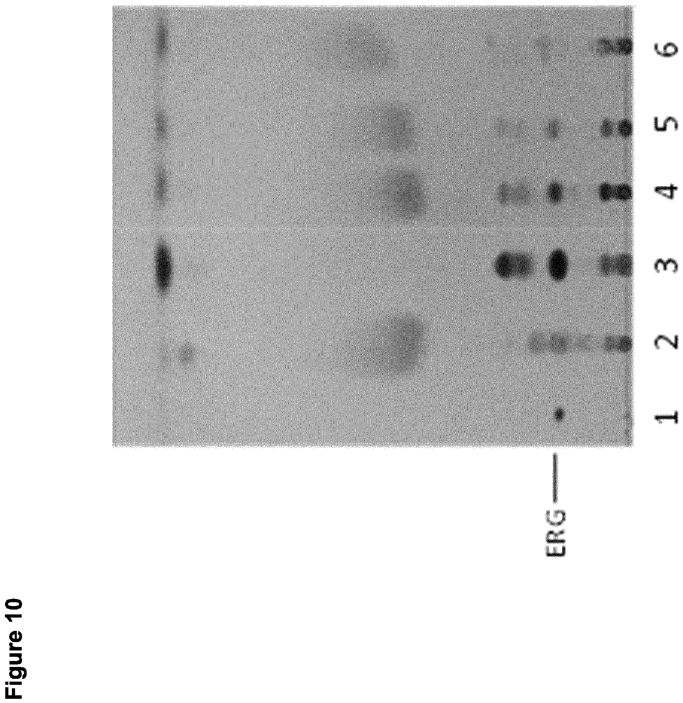

**Figure 10** shows TLC for separation of ergosterol; separation by hexane: ethyl acetate: formic acid (90:10:1, v/v/v) of ergosterol standard (line 1), yeast oil (line 2), yeast deodorizer distillate (DD)/product 6 (line 3) and three fermentation samples taken after 116 h (line 4), 146 h (line 5) and 211 h (line 6) after inoculation; ERG = ergosterol. As shown, the method of the invention allows to efficiently produce terpenoids such as ergosterol. The figure shows the fractionation and purification of ergosterol and squalene by using chromatography methods with a mobile phase of hexane: ethyl acetate: formic acid (90:10:1, v/v/v).

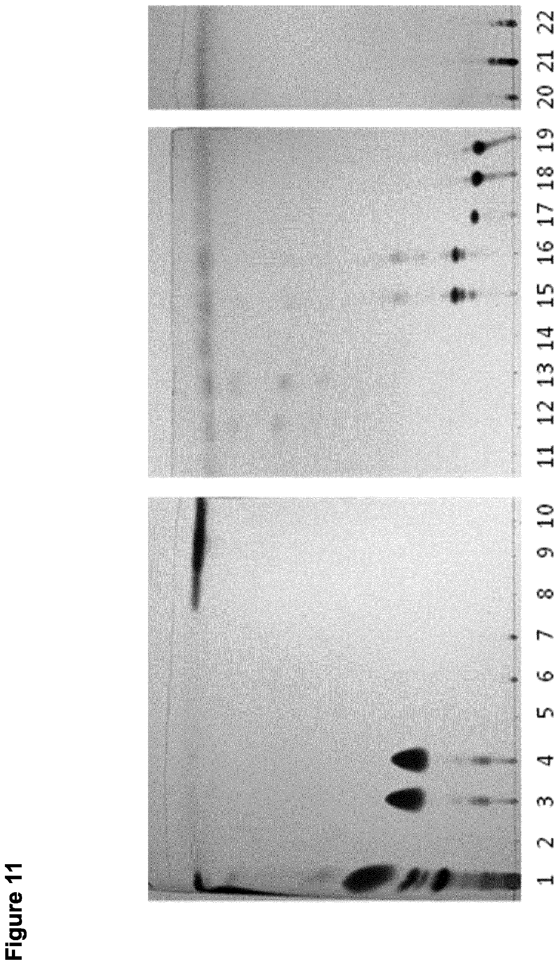

**Figure 11** shows purification of squalene and ergosterol by solid phase extraction. TLC of fractions 2 to 8 in hexane: diethyl ether: acetic acid (80:20:2, v/v/v) of Product 6 (line 1), eluate #2/3/4/5/6/7/8 of SQ standard (line 2/5/8/11/14/17/20), eluate #2/3/4/5/6/7/8 of mixture of SQ standard and Product 6 (line 3/6/9/12/15/18/21), eluate #2/3/4/5/6/7/8 of DD (line 4/7/10/13/17/19/22). The figure shows that applying different mobile phases over solid phase extraction can separate the target compounds and the hydrophobic fraction components. As demonstrated, by the method of the invention, it is possible to get purified ergosterol and squalene (purity of about 95%).

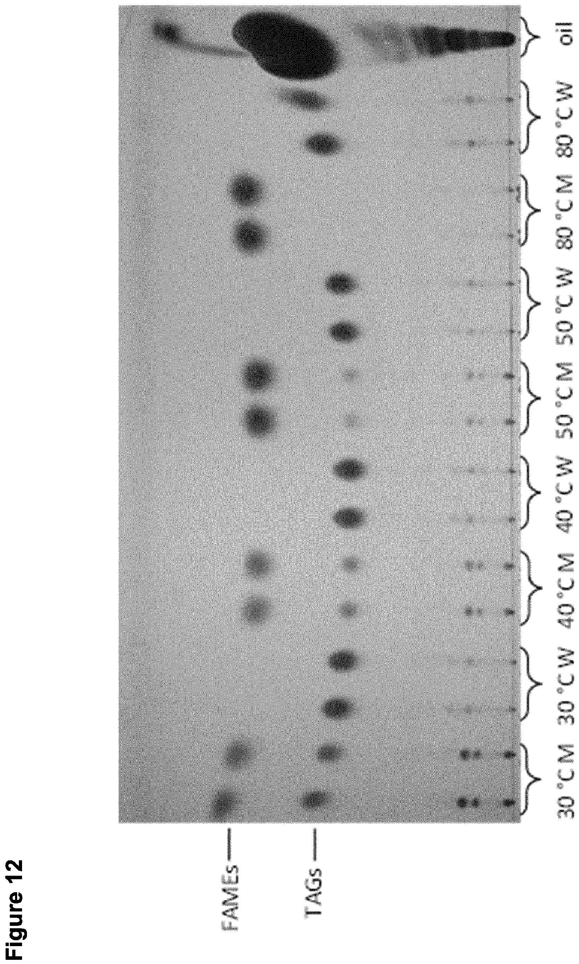

**Figure 12** shows purification of squalene and ergosterol by methylation. TLC of 30°C, 40°C, 50°C and 80°C for 5 min with double applied soft methylated Product 5, as well as not methylated Product 5 as reference (M = methylated, W = not methylated) with mobile phase hexane: diethyl ether: formic acid (v/v/v 80:20:2); FAMEs = fatty acid methyl esters, TAGs = triglycerides. The figure shows the decrease in TAGs concentration as the methylation temperature is increased. The TAGs were completely removed at 80°C (for 5 min methylation).

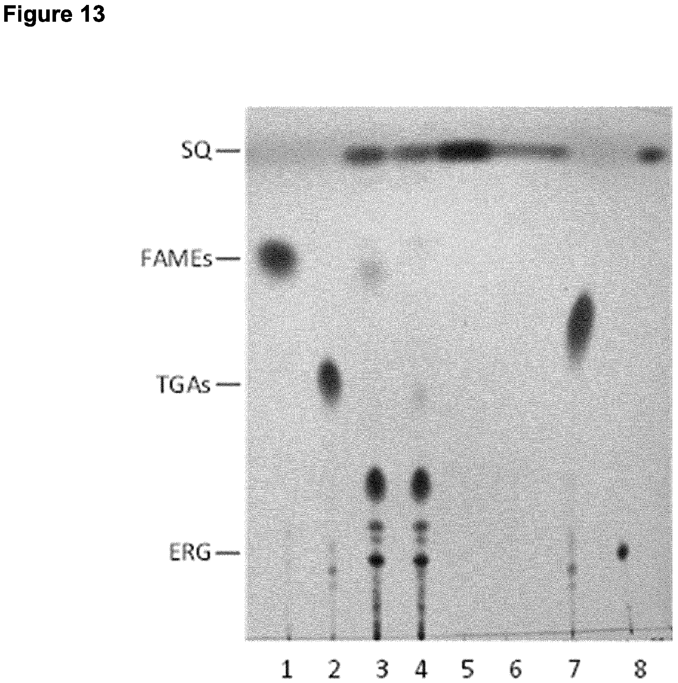

**Figure 13** shows results of the optimized soft methylation (40°C, 20 min) of Product 5, Product 6, and squalene standard; TLC with the mobile phase hexane: diethyl ether: formic acid (80:20:2, v/v/v) of Product 5 methylated (line 1), Product 5 not methylated (line 2), Product 6 methylated (line 3), Product 6 not methylated (line 4), SQ methylated (line 5), SQ not methylated (line 6), Product 5 standard (line 7) and SQ-ERG standard (line 8); SQ = squalene, FAMEs = fatty acid methyl esters, TAGs = triglycerides, ERG = ergosterol. The figure shows that soft methylation (40°C, 20 min) provides purification of the target compound in products 5 and 6 from the triglycerides; the triglycerides have converted completely to FAMEs.

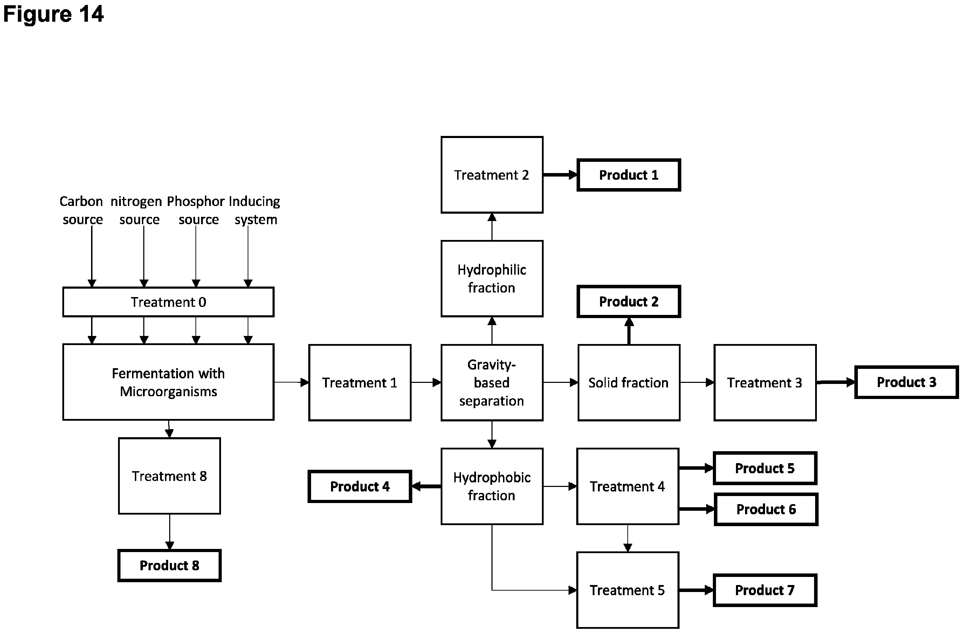

**Figure 14** shows a workflow of different embodiments of carrying out the present invention.

For example, a method of the invention can be performed to obtain any of products 1-7, as shown. The method of the invention comprises providing at least one yeast cell, growing said at least one yeast cell, and harvesting at least one target compound; however, the method may comprise further steps such as one or more of the following steps:
For example, a method of the invention may comprise a treatment step, as herein referred to as "treatment 0", for example a step of pretreating a substrate, comprising any of mechanical pretreatment, dissolving said substrate(s) in a dissolvent, chemical hydrolysis of said substrate(s), thermal pretreatment of said substrate(s), fermentative pretreatment of said substrate(s), and enzymatic pretreatment of said substrate(s) using one or more enzymes.

For example, a method of the invention may comprise a treatment step, as herein referred to as "treatment 1", which is a step of lysing said at least one yeast cell, e.g. comprising any of an enzymatic hydrolysis, temperature shock, chemical treatment, high-pressure homogenization, ultrasound homogenization, and any combination thereof; preferably comprising an enzymatic hydrolysis.

For example, a method of the invention may comprise a treatment step, as herein referred to as "treatment 2", comprising any of affinity-based separation, organic solvent extraction, ionic-liquid extraction, supercritical fluid extraction, liquid-liquid extraction, solid phase extraction, flash extraction, and any combination thereof.

For example, a method of the invention may comprise a treatment step, as herein referred to as "treatment 3", comprising any of water extraction, gravity-base separation, affinity-based separation, organic solvent extraction, ionic-liquid extraction, supercritical fluid extraction, liquid-liquid extraction, solid phase extraction, flash extraction, steam extraction, and any combination thereof.

For example, a method of the invention may comprise a treatment step, as herein referred to as "treatment 4", comprising any of degumming, neutralization, bleaching, distillation under inert gas, deodorization, and any combination thereof; preferably comprising any of neutralization, bleaching, deodorization, and any combination thereof; more preferably deodorization.

For example, a method of the invention may comprise a treatment step, as herein referred to as "treatment 5", comprising any of chromatography, affinity-based separation, organic solvent extraction, ionic-liquid extraction, supercritical fluid extraction, liquid-liquid extraction, solid phase extraction, flash extraction, steam extraction, vacuum distillation, distillation under inert or noble gases, and any combination thereof.

In the following, reference is made to the examples, which are given to illustrate, not to limit the present invention.

### EXAMPLES

### Example 1: Triterpenoids, e.g. squalene

Squalene was analyzed according to same method used for sterols. First, 5.0 g of single cell oil was spiked with 1 mL of internal standard solution of 5α-cholestane (1 mg/mL) and saponified under reflux for 1 h, in the dark, under argon, using 70 mL of ethanolic 1 M KOH. After addition of 100 mL of distilled water, the unsaponifiable compounds were extracted three times with 100 mL of diethyl ether. Combined organic phases were washed with 40 mL of distilled water, 40 mL of 0.5 M KOH solution, and several times with distilled water to reach a neutral pH value. After the neutralisation, the diethyl ether phase was evaporated to dryness by vacuum rotary evaporator (Buchi Laboratortechnik, Flawil, Switzerland). Residual free fatty acids were removed by solid-phase extraction on aminopropyl columns (LC-NH2 SPE tubes, 3 mL, 500 mg). The isolated unsaponifiable matter was dissolved in 2 ml of diethyl ether and transferred into vial for GC analysis. Analyses of target compounds were performed on GC-MS and gas chromatograph 8890 N instrument (Agilent Technologies) coupled with flame ionization detector (Figure 1). Squalene was separated on a 30 m HP-5MS column (Agilent) using a temperature gradient of 80 °C increased to 320 °C at 15 °C/min, held for 20 min. Correction factors for the internal standard and compounds of our interest were used before conversion of peak areas into mass percentages (Figure 2). A high content of squalene was achieved with the method of producing squalene using *Cutaneotrichosporon oleaginosus.*

### Example 2: Triterpenoids, e.g. sterols such as ergosterol and ergosterol derivatives, in product 4 and 5 with different Y temperatures

Ergosterol derivatives, such as provitamin D2, and its degradation products were analyzed according to standard methods used for sterols. First, 5.0 g of single cell oil was spiked with 1 mL of internal standard solution of 5α-cholestane (1 mg/mL) and saponified under reflux for 1 h, in the dark, under argon, using 70 mL of ethanolic 1M KOH. After addition of 100 mL of distilled water, the unsaponifiable compounds were extracted three times with 100 mL of diethyl ether. Combined organic phases were washed with 40 mL of distilled water, 40 mL of 0.5 M KOH solution, and several times with distilled water to reach a neutral pH value. After the neutralisation, the diethyl ether phase was evaporated to dryness by vacuum rotary evaporator (Buchi Laboratortechnik, Flawil, Switzerland). Residual free fatty acids were removed by solid-phase extraction on aminopropyl columns (LC-NH2 SPE tubes, 3 mL, 500 mg). The isolated unsaponifiable matter was dissolved in 2 ml of diethyl ether and transferred into vial for GC analysis. Analyses of target compounds were performed on GC-MS and gas chromatograph 8890 N instrument (Agilent Technologies) coupled with flame ionization detector. Ergosterol derivatives and their degradation products were separated on a 30 m HP-5MS column (Agilent) using a temperature gradient of 80 °C increased to 320 °C at 15 °C/min, held for 20 min. Correction factors for the internal standard and compounds of our interest were used before conversion of peak areas into mass percentages (Figure 3).

### Example 3: Tetraterpenoids, e.g. carotenoids and ergostatetraene, in product 4 and 5 with different X temperatures

Tetraterpenoids, such as carotenoids and ergostatetraene, were analyzed according to standard methods used for sterols, as described above.

### Example 4: Phenolic compounds, e.g. tocopherols and tocotrienols such as α-tocopherol and α-tocotrienol, in product 4 and 5 with different X temperatures

### Determination of tocochromanol content

Determination of vitamin E derivatives was done by HPLC-FLD method based on CSN EN ISO 9936 (588723) method. HPLC analysis with fluorescence detection (FLD) was performed on Agilent 1100 Series (Agilent Technologies) fitted with a Lichrospher 100 Diol column (4.0 mm i.d.; 250 mm; particle size 5 µm) (Merck Millipore) maintained at 25.0 °C. Elution was performed at a flow rate of 1.0 mL/min, using THF/heptane (96:4, v/v) as the mobile phase. Separation was achieved by isocratic flow for 45 min. The wavelengths of excitation and emission were 295 and 330 nm, respectively.

High contents of tocochromanols, particularly of α-tocopherol and α-tocotrienol, were achieved (Figure 5).

### Example 5: Products of single cell oil

Products of single cell oil were obtained using *Cutaneotrichosporon oleaginosus.* The single cell oil products comprised compounds in the following concentration ranges:

**Exemplary composition 1:**

| Ingredient | range in wt% |
|---|---|
| Triglycerides | 80 - 92 |
| Squalene | 0.1 - 5 |
| Ergosterol | 0.5 - 10 |
| Free Fatty Acids | 0.1 - 0.5 |
| Phospholipids/Sphingolipids/Ceramides | 0.01 - 0.2 |
| β-carotenes | 0.2 - 10 |
| Tocopherol and tocotrienols, | 0.1 - 5 |

**Exemplary composition 2:**

| Ingredient | range in wt% |
|---|---|
| Triglycerides | 80 - 92 |
| Terpenoid | 3.0 - 40 |
| Phenolic compound | 0.5-5 |
| Free Fatty Acids | 0.1 - 0.5 |
| Phospholipids/Sphingolipids/Ceramides | 0.01 - 0.2 |

### Example 6: Production of Squalene and Ergosterol over fermentation time

*Cutaneotrichosporon oleaginosus* was cultured. Particularly, fermentation was carried out at scale of 3 L at a temperature of 28°C and pH 6.5. Dissolved Oxygen (DO) 50% controlled by increasing the stirrer from 200 -800 rpm, aeration 0.5 -1.5 vvm, and the oxygen content in the in gas from 21 -100%. The fermentation was run for 40 h and samples were taken at 18, 30 and 40 h.

Fermentation was carried out at scale of 3 L at a temperature of 28°C and pH: 6.5. Dissolved Oxygen (DO): 25- 60, stirrer: 200 -800 Rpm, and aeration: 0.5 -1.5 vvm.

The cultivation of *Cutaneotrichosporon oleaginosus* provided a high yield of squalene and ergosterol (Figure 6). The cells were washed and then homogenized with a high pressure homogenizer. The target compounds were extracted by a solvent mix chloroform:methanol in a ratio of 2:1, v:v. Then, the same protocol as described in example 1 was followed.

### Example 7: Comparison of squalene titer and productivity between a method of the invention and previous works.

The yields of the method of the invention were compared with prior art methods involving microorganisms such as *Thraustochytrium, Pseudozyma sp., Schizochytrium mangrovei, Yarrowia lipolytica,* and *Saccharomyces cerevisiae.* Advantageously, the method of the invention achieved very high yields. Particularly, the target compound(s) such as squalene were efficiently produced such that a final concentration of 10.0-40 g/L was obtained. In contrast thereto, the prior art methods only achieved a concentration in the range of from 0.15 to 2.011 g/L. Furthermore, the method of the invention surprisingly showed exceptional productivity; particularly, the target compound was efficiently produced in an amount in the range of 0.1 - 0.45 g/L/h. In contrast thereto, the prior art methods only achieved 0.001-0.039 g/L/h.

Accordingly, the method of the invention has a higher efficiency in producing target compounds than previous methods. The exceptional productivity of the method of the invention is highly advantageous.

### Example 8: Product analysis

### Product 2 "Solid Fraction "

**Product A**

| Ingredient | range % w/w |
|---|---|
| Solid biomass | 50-92 |
| Acylglycerols | 10 - 30 |
| Squalene | 0.1 - 20 |
| Ergosterol | 0.5 - 20 |
| Free Fatty Acids | 0.1- 0.5 |
| Phospholipids/Sphingolipids/Ceramides | 0.5 - 10 |
| β-carotenes | 0.2 - 3 |
| Tocopherol and tocotrienols, | 0.1 - 3 |

**Product B**

| Ingredient | range % w/w |
|---|---|
| Solid biomass | 50-92 |
| Acylglycerols | 10 - 30 |
| Terpenoid | 3.0 - 50 |
| Phenolic compound | 0.5-5 |
| Free Fatty Acids | 0.1- 0.5 |
| Phospholipids/Sphingolipids/Ceramides | 0.5 - 10 |

### Product 3 "Treatment 3-Solid Fraction "

**Product A**

| Ingredient | range % w/w |
|---|---|
| Solid biomass | 0-1 |
| Acylglycerols | 20 - 40 |
| Squalene | 0.1 - 35 |
| Ergosterol | 0.5 - 40 |
| Free Fatty Acids | 0.1- 0.5 |
| Phospholipids/Sphingolipids/Ceramides | 0.5 - 20 |
| β-carotenes | 0.2 - 8 |
| Tocopherol and tocotrienols, | 0.1-3 |

**Product B**

| Ingredient | range % w/w |
|---|---|
| Solid biomass | 0-1 |
| Acylglycerols | 20 - 40 |
| Terpenoid | 5.0 - 60 |
| Phenolic compound | 0.5 - 10 |
| Free Fatty Acids | 0.1- 0.5 |
| Phospholipids/Sphingolipids/Ceramides | 0.5 - 20 |

### Product 4 "Crude Oil"

**Product A**

| Ingredient | range % w/w |
|---|---|
| Acylglycerols | 80 - 92 |
| Squalene | 0.1 - 20 |
| Ergosterol | 0.5 - 20 |
| Free Fatty Acids | 0.1- 0.5 |
| Phospholipids/Sphingolipids/Ceramides | 0.01 - 0.2 |
| β-carotenes | 0.2 - 10 |
| Tocopherol and tocotrienols, | 0.1-5 |

**Product B**

| Ingredient | range % w/w |
|---|---|
| Acylglycerols | 80 - 92 |
| Terpenoid | 3.0 - 50 |
| Phenolic compound | 0.5 - 5 |
| Free Fatty Acids | 0.1- 0.5 |
| Phospholipids/Sphingolipids/Ceramides | 0.01 - 1 |

### Product 5 "Refined Oil"

**Product A**

| Ingredient | range % w/w |
|---|---|
| Acylglycerols | 80 - 95 |
| Squalene | 0.1 - 10 |
| Ergosterol | 0.5 - 25 |
| Free Fatty Acids | 0.01 - 0.05 |
| Phospholipids/Sphingolipids/Ceramides | 0.01 - 0.02 |
| β-carotenes | 0.2-5 |
| Tocopherol and tocotrienols, | 0.01 - 4 |

**Product B**

| Ingredient | range % w/w |
|---|---|
| Acylglycerols | 80 - 92 |
| Terpenoid | 3.0 - 40 |
| Phenolic compound | 0.5-5 |
| Free Fatty Acids | 0.01 - 0.05 |
| Phospholipids/Sphingolipids/Ceramides | 0.01 - 0.02 |

### Product 6 "Treatment 4 distillate"

**Product A**

| Ingredient | range % w/w |
|---|---|
| Acylglycerols | 5-15 |
| Squalene | 10 - 40 |
| Ergosterol | 0.5 - 50 |
| Free Fatty Acids | 5-15 |
| Phospholipids/Sphingolipids/Ceramides | 0.01 - 0.02 |
| β-carotenes | 0.05- 15 |
| Tocopherol and tocotrienols, | 0.01 - 15 |

**Product B**

| Ingredient | range % w/w |
|---|---|
| Acylglycerols | 80 - 92 |
| Terpenoid | 10 - 65 |
| Phenolic compound | 0.05 - 15 |
| Free Fatty Acids | 5-15 |
| Phospholipids/Sphingolipids/Ceramides | 0.01 - 0.02 |

### Example 9: Product 4

*Cutaneotrichosporon oleaginosus* (ATCC 20509) was cultivated in 1-L Bioreactor: DASGIP^{®} parallel bioreactor System (Eppendorf, Germany) with a working volume of 4 times 1 L of corresponding media. The temperature was varied 28-30 °C, and the pH of the bioreactor was adjusted to pH 7.5-6.5 ± 0.2 with 1 M NaOH or 100% acetic acid or mixture of acetic acid with other organic acid. Stirring (100 - 1000 rpm), Oxygen ratio (21 - 100%) and aeration (0.5 - 2.0 vvm of air) were regulated automatically to maintain dissolved oxygen at above of pO2≥ 50%. Foam was prevented by the addition of 0.01% (V/V) of an antifoam agent (Antifoam 204, Sigma Aldrich).

Media 1: glucose 30 g L⁻¹, yeast extract 0.5 g L⁻¹, peptone 5 g.L⁻¹, sodium acetate 4.1 g L⁻¹, NH₄Cl 0.5 g L⁻¹, KH₂PO₄ 2.4 g L⁻¹, Na₂HPO₄.12H₂O 0.9 g L⁻¹, MgSO₄.7H2O 1.5 g L⁻¹, FeCl₃.6H₂O 0.08 g L⁻¹, ZnSO₄.7H₂O 0.01 g L⁻¹, CaCl₂.2H₂O 0.1 g L⁻¹, MnSO₄.5H₂O 0.1 mg L⁻¹, CuSO₄.5H₂O 0.1 mg L⁻¹, Co(NO₃)₂.6H₂O 0.1 mg L⁻¹. The bioreactor was fed by 30% acetic acid for 60 h.

| Ingredient | Amount |
|---|---|
| Acylglycerols | 97 % w/w |
| Squalene | 4.65 g/kg |
| Ergosterol+ Derivatives | 4.5 g/kg |
| Phenolic compound | **0.1** g/kg |
| Free Fatty Acids | 2 % w/w |
| Phospholipids/Sphingolipids/Ceramides | 0.5 % w/w |

### Example 10: Product 4

*Cutaneotrichosporon oleaginosus* (ATCC 20509) was cultivated in 1-L Bioreactor: DASGIP^{®} parallel bioreactor System (Eppendorf, Germany) with a working volume of 4 times 1 L of corresponding media. The temperature was varied 28-30 °C, and the pH of the bioreactor was adjusted to pH 7.5-6.5 ± 0.2 with 1 M NaOH or 100% acetic acid or mixture of acetic acid with other organic acid. Stirring (100 - 1000 rpm), Oxygen ratio (21 - 100%) and aeration (0.5 - 2.0 vvm of air) were regulated automatically to maintain dissolved oxygen at above of pO2≥ 50%. Foam was prevented by the addition of 0.01% (V/V) of an antifoam agent (Antifoam 204, Sigma Aldrich).

Media 1: glucose 30 g L⁻¹, yeast extract 1.5 g L⁻¹, peptone 3 g.L⁻¹, sodium acetate 4.1 g L⁻¹, NH₄Cl 0.5 g L⁻¹, KH₂PO₄ 2.4 g L⁻¹, Na₂HPO₄.12H₂O 0.9 g L⁻¹, MgSO₄.7H2O 1.5 g L⁻¹, FeCl₃.6H₂O 0.08 g L⁻¹, ZnSO₄.7H₂O 0.01 g L⁻¹, CaCl₂.2H₂O 0.1 g L⁻¹, MnSO₄.5H₂O 0.1 mg L⁻¹, CuSO₄.5H₂O 0.1 mg L⁻¹, Co(NO₃)₂.6H₂O 0.1 mg L⁻¹. The bioreactor was fed by 50% acetic acid and 2% glucose for 40 h.

| Ingredient | Amount |
|---|---|
| Acylglycerols | 96% w/w |
| Squalene | 5.2 g/kg |
| Ergosterol+ Derivatives | 5.1 g/kg |
| Phenolic compound | 0.1 g/kg |
| Free Fatty Acids | 2% w/w |
| Phospholipids/Sphingolipids/Ceramides | 0.5 % w/w |

### Example 11: Product 4 -

*Cutaneotrichosporon oleaginosus* (ATCC 20509) was cultivated in 1-L Bioreactor: DASGIP^{®} parallel bioreactor System (Eppendorf, Germany) with a working volume of 4 times 1 L of corresponding media. The temperature was varied 28-30 °C, and the pH of the bioreactor was adjusted to pH 7.5-6.5 ± 0.2 with 1 M NaOH or 100% acetic acid or mixture of acetic acid with other organic acid. Stirring (100 - 1000 rpm), Oxygen ratio (21 - 100%) and aeration (0.5 - 2.0 vvm of air) were regulated automatically to maintain dissolved oxygen at above of pO2≥ 50%. Foam was prevented by the addition of 0.01% (V/V) of an antifoam agent (Antifoam 204, Sigma Aldrich).

Media 1: glucose 30 g L⁻¹, yeast extract 1.5 g L⁻¹, peptone 3 g.L⁻¹, sodium acetate 4.1 g L⁻¹, NH₄Cl 0.5 g L⁻¹, KH₂PO₄ 2.4 g L⁻¹, Na₂HPO₄.12H₂O 0.9 g L⁻¹, MgSO₄.7H2O 1.5 g L⁻¹, FeCl₃.6H₂O 0.08 g L⁻¹, ZnSO₄.7H₂O 0.01 g L⁻¹, CaCl₂.2H₂O 0.1 g L⁻¹, MnSO₄.5H₂O 0.1 mg L⁻¹, CuSO₄.5H₂O 0.1 mg L⁻¹, Co(NO₃)₂.6H₂O 0.1 mg L⁻¹. The bioreactor was fed by 50% acetic acid and 2% glucose, and 1% YP Media for 40 h.

| Ingredient | Amount |
|---|---|
| Acylglycerols | 96% w/w |
| Squalene | 2.0 g/kg |
| Ergosterol+ Derivatives | 6.0 g/kg |
| Phenolic compound | 0.1 g/kg |
| Free Fatty Acids | 2.5% w/w |
| Phospholipids/Sphingolipids/Ceramides | 0.4 % w/w |

### Example 12: Product 4

*Cutaneotrichosporon oleaginosus* (ATCC 20509) was cultivated in 1-L Bioreactor: DASGIP^{®} parallel bioreactor System (Eppendorf, Germany) with a working volume of 4 times 1 L of corresponding media. The temperature was varied 28-30 °C, and the pH of the bioreactor was adjusted to pH 7.5-6.5 ± 0.2 with 1 M NaOH or 100% acetic acid or mixture of acetic acid with other organic acid. Stirring (100 - 1000 rpm), Oxygen ratio (21 - 100%) and aeration (0.5 - 2.0 vvm of air) were regulated automatically to maintain dissolved oxygen at above of pO2≥ 50%. Foam was prevented by the addition of 0.01% (V/V) of an antifoam agent (Antifoam 204, Sigma Aldrich).

Media 1: glucose 30 g L⁻¹, yeast extract 1.5 g L⁻¹, peptone 3 g.L⁻¹, sodium acetate 4.1 g L⁻¹, NH₄Cl 0.5 g L⁻¹, KH₂PO₄ 2.4 g L⁻¹, Na₂HPO₄.12H₂O 0.9 g L⁻¹, MgSO₄.7H2O 1.5 g L⁻¹, FeCl₃.6H₂O 0.08 g L⁻¹, ZnSO₄.7H₂O 0.01 g L⁻¹, CaCl₂.2H₂O 0.1 g L⁻¹, MnSO₄.5H₂O 0.1 mg L⁻¹, CuSO₄.5H₂O 0.1 mg L⁻¹, Co(NO₃)₂.6H₂O 0.1 mg L⁻¹. The bioreactor was fed by 90% acetic acid for 40 h.

| Ingredient | Amount |
|---|---|
| Acylglycerols | 96% w/w |
| Squalene | 7.0 g/kg |
| Ergosterol+ Derivatives | 4.0 g/kg |
| Phenolic compound | 0.1 g/kg |
| Free Fatty Acids | 1% w/w |
| Phospholipids/Sphingolipids/Ceramides | 0.3 % w/w |

### Example 13: Product 4

*Cutaneotrichosporon oleaginosus* (ATCC 20509) was cultivated in 1-L Bioreactor: DASGIP^{®} parallel bioreactor System (Eppendorf, Germany) with a working volume of 4 times 1 L of corresponding media. The temperature was varied 28-30 °C, and the pH of the bioreactor was adjusted to pH 7.5-6.5 ± 0.2 with 1 M NaOH or 100% acetic acid or mixture of acetic acid with other organic acid. Stirring (100 - 1000 rpm), Oxygen ratio (21 - 100%) and aeration (0.5 - 2.0 vvm of air) were regulated automatically to maintain dissolved oxygen at above of pO2≥ 50%. Foam was prevented by the addition of 0.01% (V/V) of an antifoam agent (Antifoam 204, Sigma Aldrich).

Media 1: glucose 30 g L⁻¹, yeast extract 1.5 g L⁻¹, peptone 3 g.L⁻¹, , NH₄Cl 0.5 g L⁻¹, KH₂PO₄ 2.4 g L⁻¹, Na₂HPO₄.12H₂O 0.9 g L⁻¹, MgSO₄.7H2O 1.5 g L⁻¹, FeCl₃.6H₂O 0.08 g L⁻¹, ZnSO₄.7H₂O 0.01 g L⁻¹, CaCl₂.2H₂O 0.1 g L⁻¹, MnSO₄.5H₂O 0.1 mg L⁻¹, CuSO₄.5H₂O 0.1 mg L⁻¹, Co(NO₃)₂.6H₂O 0.1 mg L⁻¹. The bioreactor was fed by 50% acetic acid for 40 h.

| Ingredient | Amount |
|---|---|
| Acylglycerols | 96% w/w |
| Squalene | 3.0 g/kg |
| Ergosterol+ Derivatives | 6.6.0 g/kg |
| Phenolic compound | 0.1 g/kg |
| Free Fatty Acids | 1%w/w |
| Phospholipids/Sphingolipids/Ceramides | 0.3 % w/w |

### Example 14: Product 5 and product 6

Two-stage laboratory degumming of hydrophobic fraction (Product 4) (14 litres, 12 kg) was performed directly in a canister with a sample previously tempered to 70 °C. Non-hydratable phospholipids were removed in the first stage by addition of 75% H₃PO₄ (0.1 %; w/w) at 70 °C. Effective distribution of phosphoric acid was done by high shear mixing. Silverson's L4RT (Silverson Machines, Inc.) high shear rotor/stator laboratory mixer was operated at 2500 min-1. Distilled water (3 %; w/w) was added after 7 min retention under slow stirring (70 min-1) and agglomerated gums were allowed to settle down.

Second degumming stage was performed in the same manner with distilled water (4 %, w/w; retention 5 min) only. Phosphorus content was monitored during two-stage degumming process. Standard deeply degummed hydrophobic fraction (14 litres, 12 kg) was alkali neutralized by solution of sodium hydroxide (5%). The lye solution was dosed equimolarly based on free fatty acid content. Both, the sample and the lye solution were tempered to 70 °C. Effective distribution of lye solution was done by high shear mixing. Silverson's L4RT (Silverson Machines, Inc.) high shear rotor/stator laboratory mixer was operated at 1500 min-1. Distilled water (3 %; w/w) was added after 7 min retention under slow stirring (70 min-1) and agglomerated soapstock was allowed to settle down. Afterwards, alkali neutralized hydrophobic fraction was thoroughly washed by distilled water until neutral pH. Hydrophobic fraction freed of organic acids was dried prior adsorptive bleaching by Na2SO₄. Adsorptive bleaching was done as follows. Dried hydrophobic fraction sample was pre-heated to 50 °C and loaded into the glass apparatus through which a stream of argon passed upwards (0.2 - 1.0 ml Ar/ml of hydrophobic fraction per min) in order to remove the remaining air and to agitate the oil sample properly. The batch was subsequently heated up, particularly to a bleaching temperature of 90 °C (bleached), 110°C (refined I), or 120°C (refined II), and the bleaching clay Tonsil Optimum 210 FF (2 %; w/w) was added. The stream of argon was increased to 2.0 - 4.0 ml of Ar/ml of hydrophobic fraction per min during the adsorptive bleaching process. Laboratory bleaching was finished after 50 min and half-refined hydrophobic fraction was filtrated after cooling down to 70 °C.

Two techniques were employed for the separation of terpenes and phenolic compounds. In the first experimental series, laboratory deodorization-deacidification was applied Standard half-refined degummed, neutralized and bleached hydrophobic fraction was pre-heated to 100 °C and loaded into the glass apparatus through which a stream of argon passed upwards (0.5 - 1.0 ml of Ar/ml of hydrophobic fraction per min). The batch was subsequently heated up to a deodorisation temperature of 230 °C and the stream of the inert gas was increased to 2.0 - 4.0 ml of Ar/ml of hydrophobic fraction per minute. Temperature was increased by 10 °C each 30/60 minutes to simulate deodoriser tray. Deodorisation operation time was 90 minutes under a vacuum of 2 hPa. The adapted apparatus was connected to a vacuum pump with high performance. The stream of the inert gas passing through the layer of hydrophobic fraction was stabilised. It was observed that Ar bubbles increased in volume from 10 to 21 µm during the isothermal movement through the layer of hydrophobic fraction. At the given intervals, aliquots were taken for the analysis.

In the second experimental series, short path distillation experiments were carried out within Pilodist SP500 lab scale equipment (PILODIST GmbH) with thermostat mantle and internal condenser The system allowed gentle processing of thermally sensible hydrophobic fraction with following parameters: distillation residue temperature (80°C); condensate temperature (30°C); operating pressure (1 hPa); feed hydrophobic fraction temperature (Y°C); stirring rate: 300 min-1.

*Description of product 6, where X is 90°C and Y is 80°C.*

| Ingredient | Amount |
|---|---|
| Acylglycerols | 7 % w/w |
| Squalene | 29 % w/w |
| Ergosterol+ Derivatives | 42 % w/w |
| Phenolic compound | 4 g/kg |
| Free Fatty Acids | 18 % w/w |
| Phospholipids/Sphingolipids/Ceramides | 0.1 % w/w |

### Example 15: Triterpenoids - Sterols - Ergosterol and Ergosterol derivatives in product 6 and 5 with different Y temperatures

In the second experimental series, short path distillation experiments were carried out within Pilodist SP500 lab scale equipment (PILODIST GmbH) with thermostat mantle and internal condenser. The system allowed gentle processing of thermally sensible hydrophobic fraction with following parameters: distillation residue temperature (80°C); condensate temperature (30°C); operating pressure (1 hPa); feed hydrophobic fraction temperature (Y°C); and stirring rate: 300 min-1. The abbreviation "deo" means deodorization on the short path distillation equipment at the given temperature (Y: 210 - 230 °C) with a constant pressure of 1 mbar.

### Example 16: Composition of product 6 from treatment 4 compared to other oils The experiment was performed as described for example 14. X was 100°C and Y was 230°C.

| | Soya oil | Canola oil | Sunflower oil | Palm oil | Our method |
|---|---|---|---|---|---|
| FFA (%) | 25-40 | | | 85-90 | 55.4 |
| Tocopherols (%) | 5-20 | 8 | 5-10 | 0.15-0.30 | 0.6 |
| Sterols (%) | 6-23 | 7 | 12-24 | 0.2-0.4 | 13 |
| Squalene (%) | 0.1-3.0 | - | 0.5 | 0.5-1.0 | 16 |
| Neutral lipid (%) | 10-50 | | | 6-9 | 15 |
| Neutral lipid = mono- + di- + triglycerides; 2expressed FFA= free fatty acids | | | | | |

### Example 17: Purification of squalene and ergosterol by chromatography-based methods (treatment 6)

In order to achieve a good separation with respect to the detection of squalene and ergosterol in Product 5 and Product 6, it is necessary to find a suitable solvent composition for the used mobile phase. Depending on the polarity of the mobile phase, samples can be separated in different directions. This enables a resolution of the various components.

The use of a mobile phase consisting of hexane: diethyl ether: acid (80:20:2, v/v/v) was shown to allow the separation shift to take place on the basis of the used acid. Thus, the stained TLCs of Product 5 (line 1) and Product 6 (line 2) are shown Figure 8 in using three different acids in the mobile phases: formic acid (Figure 8 (D)), acetic acid (Figure 8 (C)) and propionic acid (Figure 8 (B)). By using all three mobile phase compositions, a successful separation of the samples was achieved. The triglycerides as well as other components with a lower proportion are clearly visible in all TLCs of the Product 5 (Figure 8 (B, C, D)). In contrast, the separation of Product 6 exhibits high levels of FFA, SQ, and SE, as well as many other constituents that have transferred from the crude oil to the distillate during the refining process.

When using the strongest acid, formic acid (Figure 8 (D)), improved separation of the polar components of the samples takes place. In the opposite, the mobile phase with propionic acid (Figure 25 (B)) shows a separation in the more nonpolar region of the sample. The use of the mobile phase with acetic acid (Figure 8 (C)) is a good running medium for screening the entire sample. This is because it provides a relatively uniform separation of the complete sample. Utilizing a similar mobile phase composition (n-hexanes: diethyl ether: acetic acid (85:15:1, v/v/v)), others have also achieved separation of lipids as shown in Figure 8 (A). When comparing Figure 8 (A) to Figure 8 (B) and (C), a very similar separation of the hydrophobic fraction can be seen. This allows individual components to be identified, like TGAs, FFA, SQ and SE. Based on the applied mobile phases and their different compositions, the separation of Product 5 and Product 6 could be successfully carried out.

### REFERENCES

[1] Patel, L. Mu, Y. Shi, U. Rova, P. Christakopoulos, and L. Matsakas, Novel Biorefinery Approach Aimed at Vegetarians Reduces the Dependency on Marine Fish Stocks for Obtaining Squalene and Docosahexaenoic Acid, ACS Sustainable Chemistry & Engineering 8: 23 (2020) 8803-8813.

The features of the present invention disclosed in the specification, the claims, and/or in the accompanying figures may, both separately and in any combination thereof, be material for realizing the invention in various forms thereof.

## Claims

1. A method for producing at least one target compound, said method comprising the steps:
a) providing at least one yeast cell, wherein said yeast cell is a yeast from the family Trichosporonaceae; wherein, preferably, said yeast is from the genus *Cutaneotrichosporon;*
b) growing said at least one yeast cell in a medium comprising a carbon source, a nitrogen source, and a phosphate source, and thereby allowing said at least one yeast cell to produce at least one target compound;
c) optionally, lysing said at least one yeast cell;
d) harvesting said at least one target compound;
e) optionally, purifying said at least one target compound;
wherein said at least one target compound is selected from a terpenoid, a phenolic compound, a vitamin, and a combination thereof.

2. The method according to claim 1, wherein said yeast is selected from the group consisting of *Aegeritella sp., Apiotrichum sp., Cutaneotrichosporon sp.,* and *Trichosporon sp.,* preferably selected from *Cutaneotrichosporon sp.,* more preferably is *Cutaneotrichosporon oleaginosus.*

3. The method according to claim 1 or 2, wherein said terpenoid is selected from the group consisting of monoterpenoids, sesquiterpenoids, diterpenoids, triterpenoids, and tetraterpenoids,
wherein, optionally, triterpenoids are selected from the group consisting of squalene, 2,3-epoxysqualene, sterols, ergosterol, cholesterol, cycloartenol, stigmasterol, β-sitosterol, campesterol, fucosterol, lanosterol, steroid, oleanolic acid, glycyrrhizin, glycyrrhetinic acid, ursolic acid, betulin, betulinic acid, lupeol, and derivatives thereof, preferably selected from squalene, ergosterol, and ergosterol derivatives,
wherein, optionally, tetraterpenoids are selected from the group consisting of phytoene, xanthophylls, acyclic lycopene, and carotenoids, preferably selected from monocyclic γ-carotene and bicyclic α- and β-carotenes, more preferably selected from β-carotenes;
wherein, preferably, said terpenoid is selected from the group consisting of triterpenoids, such as squalene and sterols, e.g. ergosterol, ergosterol derivatives, cholesterol, cycloartenol, lanosterol, secosteroid, and steroid; and tetraterpenoids, such as acyclic lycopene and carotenoid, e.g. monocyclic γ-carotene and bicyclic α- and β-carotenes.

4. The method according to any one of the foregoing claims, wherein said phenolic compound is selected from the group consisting of tocopherols, e.g. α-, β-, γ-, and δ-tocopherol; tocotrienols, e.g. α-, β-, γ-, and δ-tocotrienol; tocomonoenols, e.g. α- and β-tocomonoenol; phytoestrogens; chalcones, e.g. arbutin, phloretin, phloridzin, and chalconaringenin; flavonoids, e.g. flavonols, flavones, flavanones, flavanols, flavan-3-ols, anthocyanidins, isoflavones, and condensed tannins; and non-flavonoids, e.g. coumarins and phenolic acids;
wherein, preferably, said phenolic compound is selected from the group consisting of α-tocopherol, β-tocopherol, γ-tocopherol, δ-tocopherol, α-tocotrienol, β-tocotrienol, γ-tocotrienol, δ-tocotrienol, and α-tocomonoenol;
wherein, more preferably, said phenolic compound is selected from the group consisting of α-tocopherol and α-tocotrienol; and/or
wherein said vitamin is selected from vitamin A, vitamin B₁, vitamin B₂, vitamin B₃, vitamin B₅, vitamin B₆, vitamin B₇, vitamin B₉, vitamin B₁₂, and vitamin K.

5. The method according to any one of the foregoing claims, wherein said at least one target compound comprises a terpenoid and a phenolic compound; and/or
wherein said at least one target compound is squalene, ergosterol, an ergosterol derivative, α-tocotrienol, α-tocopherol, and/or γ-tocopherol, preferably squalene, ergosterol, α-tocotrienol, α-tocopherol, and/or γ-tocopherol.

6. The method according to any one of the foregoing claims, wherein
said carbon source is selected from the group consisting of carbohydrates; monosaccharides, e.g. mannitol, pentoses, and hexoses, preferably glucose and xylose; oligosaccharides, e.g. maltose, lactose, sucrose, cellobiose, laminarose, cellodextrins, trehalose, raffinose, and melibiose; glycerol; amino acids; fatty acids; organic acid, e.g. acetic acid, malonic acid, oxalic acid, citric acid, propionic acid, valeric acid, acrylic acid, crotonic acid, butyric acid, isobutyric acid, isovaleric acid, 3-hydroxybutyric acid, 3-hydroxypropionic acid, 2-hydroxybutyric acid, lactic acid, and the respective salt(s) of such acid; hydrolysates of animal tissues, plant tissues, industrial waste, food residues, or of microorganisms; and combinations of any of the foregoing; wherein, preferably, said carbon source is glucose, xylose, and/or acetic acid;
said nitrogen source is selected from the group consisting of organic nitrogen compounds, e.g. amines, amides, alkyl nitrates, nitrosamines, nitroarenes, and peroxyacyl nitrates; inorganic nitrogen compounds, e.g. ammonium salts, nitrate salts, and nitrite salts; amino acids; peptides; protein hydrolysates, e.g. peptone, tryptone, and other peptidic hydrolysates, wherein peptidic hydrolysates preferably comprise animal tissue, plant tissue, and/or components of said yeast; N-acetylglucosamine; and urea; preferably selected from the group consisting of ammonium salts, amino acids, peptides, N-acetylglucosamine, and urea; and/or
said phosphate source is selected from the group consisting of organic phosphate compounds, e.g. parathion, malathion, phospholipids, ATP, ADP, AMP, and organophosphate compounds; and inorganic phosphate salts, e.g. H₃PO₄, M₂HPO₄, MH₂PO₄, MHPO₄, and MPO₄, wherein M is a metal ion.

7. The method according to any one of the foregoing claims, wherein said growing in step b) comprises a fermentative cultivation of said at least one yeast cell, wherein said at least one target compound is produced as a coproduct of said fermentative cultivation; and/or
wherein said growing in step b) comprises cultivating said at least one yeast cell for a period of from 1 to 7 days, preferably of from 2 to 4 days;
at a temperature in the range of from 10 °C to 45 °C, preferably in the range of from 15 °C to 40°C, more preferably in the range of from 20 °C to 33 °C;
at a pH in the range of from 4 to 9.5, preferably in the range of from 5 to 8.5, more preferably in the range of from 5.5 to 8;
in a medium selected from minimal nitrogen media, minimal phosphate media, minimal sulfate media, and acetate rich media; and/or
with dissolved oxygen (pO₂) in a range of from 10 % to 90 %, preferably of from 20 % to 80 %, more preferably of from 30 % to 65 %.

8. The method according to any one of the foregoing claims, wherein said yeast cell is a wild type yeast cell or a genetically modified yeast cell; wherein, preferably, said genetic modification comprises untargeted genetic modification, such as a chemically induced mutation or a radiation induced mutation, and/or targeted genetic modification, such as a modification using a CRISPR-CAS system, a bacterial transformation, and/or plasmid-based insertion.

9. The method according to any one of the foregoing claims, wherein said harvesting in step d) comprises density-based separation, drying, floating, solvent-based extraction, chromatography, distillation, maceration, supercritical fluid extraction, enfleurage, press extraction, demulsification, decantation, and/or aspiration, preferably density-based separation,
wherein, optionally, said density-based separation is selected from separation based on natural gravity, gravity-assisted phase separation, and centrifugation, wherein each of said separation based on natural gravity, gravity-assisted phase separation, and centrifugation, is performed alone or in combination with a decantation, aspiration or other mechanical harvesting method.

10. The method according to any one of the foregoing claims, wherein said harvesting in step d) comprises harvesting said at least one target compound from said medium, from a produced yeast oil, e.g. from an unsaponifiable matter of a produced yeast oil, and/or from a cell debris, preferably from an unsaponifiable matter of a produced yeast oil, optionally after a step of oil extraction;
wherein, optionally, said harvesting in step d) further comprises harvesting a compound other than the at least one target compound, preferably an oil, sugar, amino acid, and/or organic acid.

11. The method according to any one of the foregoing claims, wherein said method comprises producing more than one target compound, preferably a terpenoid and a phenolic compound, more preferably at least two of squalene, ergosterol, an ergosterol derivative, α-tocotrienol, α-tocopherol, and γ-tocopherol.

12. The method according to any one of the foregoing claims, wherein said lysing said at least one yeast cell in step c) comprises enzymatic hydrolysis, temperature shock, chemical treatment, high-pressure homogenization, and/or ultrasound homogenization, preferably comprises enzymatic hydrolysis.

13. The method according to any of the foregoing claims, wherein said medium used in step b) has a weight ratio of carbon to nitrogen (C:N) < 100, more preferably ≤ 80, even more preferably in a range of from 25 to 80; and/or
has a weight ratio of carbon to phosphate (C:P) ≥ 10, more preferably ≥ 20, even more preferably in a range of from 25 to 100; or
wherein said medium used in step b) is a limited nitrogen medium with a weight ratio of carbon to nitrogen (C:N) ≥ 80, more preferably ≥ 100, even more preferably ≥ 150; and/or is a limited phosphate medium with a weight ratio of carbon to phosphate (C:P) ≥ 100, more preferably ≥ 200, even more preferably ≥ 500, and/or is a limited sulfate medium with a weight ratio of carbon to sulfate (C:S) ≥ 10, more preferably ≥ 20, even more preferably ≥ 50.

14. The method according to any one of the foregoing claims, wherein said purifying in step e) is performed using a separation method comprising chromatography, affinity-based separation, organic solvent extraction, ionic-liquid extraction, supercritical fluid extraction, liquid-liquid extraction, solid phase extraction, flash extraction, steam extraction, vacuum distillation, distillation under inactive or noble gases, and/or deodorization.

15. Composition comprising at least one target compound selected from a terpenoid, a phenolic compound, a vitamin, and a combination thereof,
wherein
the composition comprises solid biomass in a range of from 50-92 % w/w; acylglycerol(s) in a range of from 10-30 % w/w; squalene in a range of from 0.1-20 % w/w; ergosterol in a range of from 0.5-20 % w/w; free fatty acids in a range of from 0.1-0.5 % w/w; phospholipids, sphingolipids, and/or ceramides in a range of from 0.5-10 % w/w; β-carotene(s) in a range of from 0.2-3 % w/w; and tocopherol and/or tocotrienol(s) in a range of from 0.1-3 % w/w; or
the composition comprises solid biomass in a range of from 50-92 % w/w; acylglycerol(s) in a range of from 10-30 % w/w; terpenoid(s) in a range of from 3.0-30 % w/w; phenolic compound(s) in a range of from 0.5-5 % w/w; free fatty acids in a range of from 0.1- 0.5 % w/w; and phospholipids, sphingolipids, and/or ceramides in a range of from 0.5-10 % w/w; or
the composition comprises solid biomass in a range of from 0-1 % w/w; acylglycerol(s) in a range of from 20-40 % w/w; squalene in a range of from 0.1-35 % w/w; ergosterol in a range of from 0.5-40 % w/w; free fatty acids in a range of from 0.1- 0.5 % w/w; phospholipids, sphingolipids, and/or ceramides in a range of from 0.5-20 % w/w; β-carotene(s) in a range of from 0.2-8 % w/w; and tocopherol and/or tocotrienol(s) in a range of from 0.1-3 % w/w; or
the composition comprises solid biomass in a range of from 0-1 % w/w; acylglycerol(s) in a range of from 20-40 % w/w; terpenoid(s) in a range of from 5.0-60 % w/w; phenolic compound(s) in a range of from 0.5-10 % w/w; free fatty acids in a range of from 0.1-0.5 % w/w; and phospholipids, sphingolipids, and/or ceramides in a range of from 0.5-20 % w/w; or
the composition comprises acylglycerol(s) in a range of from 80-92 % w/w; squalene in a range of from 0.1-20 % w/w; ergosterol in a range of from 0.5-20 % w/w; free fatty acids in a range of from 0.1- 0.5 % w/w; phospholipids, sphingolipids, and/or ceramides in a range of from 0.01-0.2 % w/w; β-carotene(s) in a range of from 0.2-10 % w/w; and tocopherol and/or tocotrienols in a range of from 0.1-5 % w/w; or
the composition comprises acylglycerol(s) in a range of from 80-92 % w/w; terpenoid(s) in a range of from 3.0-50 % w/w; phenolic compound(s) in a range of from 0.5-5 % w/w; free fatty acids in a range of from 0.1- 0.5 % w/w; and phospholipids, sphingolipids, and/or ceramides in a range of from 0.01-1 % w/w; or
the composition comprises acylglycerol(s) in a range of from 60-92 % w/w; squalene in a range of from 0.1-10 % w/w; ergosterol in a range of from 0.5-25 % w/w; free fatty acids in a range of from 0.01-0.05 % w/w; phospholipids, sphingolipids, and/or ceramides in a range of from 0.01-0.02 % w/w; β-carotene(s) in a range of from 0.2-5 % w/w; and tocopherol and/or tocotrienols in a range of from 0.01-4 % w/w; or
the composition comprises acylglycerol(s) in a range of from 60-92 % w/w; terpenoid(s) in a range of from 3.0-40 % w/w; phenolic compound(s) in a range of from 0.5-5 % w/w; free fatty acids in a range of from 0.01-0.05 % w/w; and phospholipids, sphingolipids, and/or ceramides in a range of from 0.01-0.02 % w/w; or
the composition comprises acylglycerol(s) in a range of from 5-15 % w/w; squalene in a range of from 10-40 % w/w; ergosterol in a range of from 0.5-50 % w/w; free fatty acids in a range of from 5-15 % w/w; phospholipids, sphingolipids, and/or ceramides in a range of from 0.01-0.02 % w/w; β-carotene(s) in a range of from 0.05-15 % w/w; and tocopherol and/or tocotrienols in a range of from 0.01-15 % w/w; or
the composition of the invention comprises acylglycerol(s) in a range of from 5-15 % w/w; terpenoid(s) in a range of from 10-65 % w/w; phenolic compound(s) in a range of from 0.05-15 % w/w; free fatty acids in a range of from 5-15 % w/w; and phospholipids, sphingolipids, and/or ceramides in a range of from 0.01-0.02 % w/w.
